# EUROPEAN PATENT APPLICATION

(11) **EP 4 270 268 A1**
(43) Date of publication of application: **01.11.2023**
(21) Application number: 21915288.1
(22) Date of filing: 27.12.2021
(51) Int. Cl.: G06N 20/00, A61B 5/372

(54) **SYSTEM, METHOD, AND PROGRAM FOR ESTIMATING SUBJECTIVE EVALUATION BY ESTIMATION SUBJECT**

(30) Priority: 28.12.2020 JP 2020219129
(71) Applicant: OSAKA UNIVERSITY, Suita-shi Osaka 565-0871 (JP); PaMeLa, Inc., Suita-shi, Osaka 565-0863 (JP)
(72) Inventor: NAKAE Aya, Suita-shi, Osaka 565-0871 (JP); NOMURA Koutarou, Suita-shi, Osaka 565-0863 (JP)
(74) Representative: Ter Meer Steinmeister & Partner
(86) International application number: PCT/JP2021/048651
(87) International publication number: WO 2022/145429

(57) **Abstract**

Provided is a system for estimating a subjective evaluation by an estimation subject. This system for estimating a subjective evaluation by an estimation subject comprises: a reception means that receives feature data of a biosignal acquired from the estimation subject; a storage means that stores a plurality of feature templates extracted from a plurality of biosignals acquired from a plurality of subjects to be modeled including a first subject to be modeled and a second subject to be modeled, or that stores a plurality of models trained using said feature templates; and an estimation means that estimates a subjective evaluation by the estimation subject on the basis of the feature data and the plurality of feature templates or the plurality of models.

## Description

### TECHNICAL FIELD

The present disclosure relates to a system, a method and a program for estimating a subjective assessment made by an estimation target object. More specifically, the present disclosure relates to a system, a method and a program for estimating a pain experienced by an estimation target object.

### BACKGROUND ART

A subjective physiological assessment item such as a pain is often subjectively expressed. However, in order to perform an appropriate medical treatment or to perform work related to treatment and prevention such as pharmaceutical development, it is desired that a physiological assessment item be objectively assessed. Underestimation or overestimation of a pain often impedes an appropriate assessment and an appropriate medical treatment, whereby a patient suffers a disadvantage and pharmaceuticals are not appropriately developed in many cases. Therefore, a method for objectively estimating a pain, which is one of physiological assessment items, based on brain waves is proposed as an example (see, for example, Patent Document 1).

However, it is difficult to objectively assess the subjective physiological assessment items, including the pain, because subjective elements are involved in assessment of the intensity thereof. Further, an objective physiological assessment item, such as a brain wave signal, often does not necessarily correspond to a subjective experience.

Patent Document 1: Japanese Unexamined Patent Application (Translation of PCT Application), Publication No. 2010-523226

### DISCLOSURE OF THE INVENTION

### Means for Solving the Problems

One aspect of the present disclosure provides a system, a method and a program for estimating a subjective assessment made by an estimation target object. One embodiment of the present disclosure provides a system, a method and a program that enable a subjective assessment more efficiently using objective assessment items. More particularly, the present disclosure estimates a subjective assessment made by an estimation target object, based on feature data of a biosignal acquired from the estimation target object, a plurality of feature templates that are stored or a plurality of models that are stored.

As examples of embodiments of the present disclosure, the following are given.

### (Item 1)

A system for estimating a subjective assessment made by an estimation target object, the system including:
a reception means that receives feature data of a biosignal acquired from the estimation target object;
a storage means that stores a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, and the plurality of models including a first model that has learned the first feature template and a second model that has learned the second feature template; and
an estimation means that estimates the subjective assessment made by the estimation target object, based on the feature data and the plurality of feature templates or the plurality of models.

### (Item 2)

The system according to Item 1, wherein the estimation means is configured to perform:
obtaining a plurality of correlation coefficient sets by correlating each of the plurality of feature templates with the piece of feature data; and
estimating the subjective assessment made by the estimation target object, based on the plurality of correlation coefficient sets.

### (Item 3)

The system according to Item 2, wherein the estimating of the subjective assessment by the estimation target object based on the plurality of correlation coefficient sets includes:
identifying, for each of the plurality of correlation coefficient sets, a value indicating a subjective assessment that is associated with a sample corresponding to a highest correlation coefficient;
taking an ensemble average of the values indicating the subjective assessments of the plurality of correlation coefficient sets; and
determining a score indicating the subjective assessment based on the ensemble average.

### (Item 4)

The system according to Item 2, wherein the estimating of the subjective assessment by the estimation target object based on the plurality of correlation coefficient sets includes:
identifying, for each of the plurality of correlation coefficient sets, values indicating subjective assessments that are associated with samples corresponding to a top plurality of correlation coefficients;
obtaining an ensemble average correlation coefficient by, for each of the correlation coefficient sets, taking an ensemble average of the values indicating the top plurality of the subjective assessments;
taking an ensemble average of the ensemble average correlation coefficients of the plurality of correlation coefficient sets; and
determining a score indicating the subjective assessment based on the ensemble average.

### (Item 5)

The system according to Item 1, wherein the estimation means is configured to perform:
obtaining a plurality of outputs by inputting the feature data to each of the plurality of models, the plurality of outputs including a first output outputted from the first model and a second output outputted from the second model; and
estimating the subjective assessment made by the estimation target object, based on the plurality of outputs.

### (Item 6)

The system according to Item 5, wherein the estimating of the subjective assessment by the estimation target object based on the plurality of outputs includes:
taking an ensemble average of the plurality of outputs; and
determining a score indicating the subjective assessment based on the ensemble average.

### (Item 7)

The system according to Item 5 or 6, wherein
the storage means further stores a plurality of standardization parameters extracted from the plurality of biosignals, the plurality of standardization parameters including a plurality of first standardization parameters extracted from the first biosignal acquired from the first modeling target object and a plurality of second standardization parameters extracted from a plurality of second biosignals acquired from the second modeling target object,
the estimation means is configured to further perform
generating a plurality of pieces of standardized feature data by standardizing the feature data by the plurality of standardization parameters, the plurality of pieces of standardized feature data including a plurality of pieces of first standardized feature data obtained by standardizing the feature data by the plurality of first standardization parameters, and a plurality of pieces of second standardized feature data obtained by standardizing the feature data by the plurality of second standardization parameters, and the obtaining of the plurality of outputs by inputting the feature data to each of the plurality of models includes
obtaining a plurality of outputs of the plurality of models by inputting the plurality of pieces of standardized feature data to the plurality of models, the plurality of outputs of the plurality of models including a plurality of first outputs obtained by inputting the plurality of pieces of first standardized feature data to the first model and a plurality of second outputs obtained by inputting the plurality of pieces of second standardized feature data to the second model.

### (Item 8)

The system according to Item 7, wherein the estimating of the subjective assessment by the estimation target object based on the plurality of outputs includes:
obtaining a plurality of ensemble average outputs by taking an ensemble average of the plurality of outputs of each of the plurality of models, the plurality of ensemble average outputs including a first ensemble average output obtained by taking an ensemble average of the plurality of first outputs and a second ensemble average output obtained by taking an ensemble average of the plurality of second outputs;
taking an ensemble average of the plurality of ensemble average outputs; and
determining a score indicating the subjective assessment based on the ensemble average.

### (Item 9)

The system according to any one of Items 1 to 8, wherein
the reception means receives no-load feature data of a biosignal when a load is not given to the estimation target object; and
the estimation means is configured to perform:
   selecting at least one of the plurality of feature templates to be used to estimate the subjective assessment by the estimation target object or at least one of the plurality of models to be used to estimate the subjective assessment by the estimation target object, based on the no-load feature data; and
   estimating the subjective assessment made by the estimation target object, based on the feature data and the at least one of the plurality of the feature templates or the at least one of the plurality of models that has been selected.

### (Item 10)

The system according to any one of Items 1 to 9, wherein the plurality of modeling target objects are n modeling target objects, the plurality of feature templates are n feature templates, the plurality of models are n models, and n is an integer equal to or larger than two.

### (Item 11)

The system according to any one of Items 1 to 10, wherein
the biosignal acquired from the estimation target object is a biosignal at a time when a stimulus is given to the estimation target object;
the plurality of biosignals are a plurality of biosignals at a time when a stimulus is given to the plurality of modeling target objects, the first biosignal is a first biosignal at the time when the stimulus is given to the first modeling target object, and the second biosignal is a second biosignal at the time when the stimulus is given to the second modeling target object; and
the estimation means estimates a pain experienced by the estimation target object.

### (Item 12)

A method for estimating a subjective assessment made by an estimation target object, the method being executable by a computer system including a storage means, the storage means storing a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, the plurality of models including a first model that has learned the first feature template and a second model that has learned the second feature template, the method comprising: receiving feature data of a biosignal acquired from the estimation target object; and
estimating the subjective assessment made by the estimation target object, based on the feature data and the plurality of feature templates or the plurality of models.

### (Item 12A)

The method according to Item 12, further including a characteristic or characteristics according to any one or more of Items 1 to 11.

### (Item 13)

A program for estimating a subjective assessment made by an estimation target object, the program being executable by a computer including a processor unit, the program being executable by a computer system including a storage means, the storage means storing a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, the plurality of models including a first model that has learned the first feature template and a second model that has learned the second feature template, the program causing the processor unit to perform a process including:
receiving feature data of a biosignal acquired from the estimation target object; and
estimating the subjective assessment made by the estimation target object, based on the feature data and the plurality of feature templates or the plurality of models.

### (Item 13A)

The program according to Item 13, further including a characteristic or characteristics according to any one or more of Items 1 to 11.

### (Item 14)

A computer-readable storage medium storing the program according to Item 13 or 13A.

In the present disclosure, it is intended that one or more of the above characteristics can be further combined and provided, in addition to the expressly shown combinations. One skilled in the art recognizes further embodiments and advantages of the present disclosure by reading and understanding the detailed description below as necessary.

### Effects of the Invention

The present disclosure can provide a system, a method and a program for estimating a subjective assessment made by an estimation target object. Further, the present disclosure can highly accurately predict a subjective assessment by an estimation target object without giving a load to the estimation target object in advance. Especially, the present disclosure can highly accurately predict a pain that can be experienced by an estimation target object without giving a pain to the estimation target object in advance.

### BRIEF DESCRIPTION OF THE DRAWINGS

FIG. 1 is a diagram showing the concept of an example of an algorithm of the present disclosure;
FIG. 2 is a diagram showing the concept of another example of the algorithm of the present disclosure;
FIG. 3 is a diagram showing an example of the configuration of a system 100 for estimating a subjective assessment made by an estimation target object;
FIG. 4 is a diagram showing the concept of a method for creating standardization parameters;
FIG. 5 is a diagram showing an example of a process 500 by the system 100 for estimating a subjective assessment made by an estimation target object;
FIG. 6 is a diagram of an example of a process 600 for estimating a pain experienced by an estimation target object by the system 100 for estimating a subjective assessment made by an estimation target object;
FIG. 7 is a temperature profile used as a stimulus in examples 1 and 2;
FIG. 8 is a temperature profile used as a stimulus in the examples 1 and 2;
FIG. 9A is a diagram showing a result in the example 1;
FIG. 9B is a diagram showing a result in the example 1;
FIG. 10A is a diagram showing a result in the example 2;
FIG. 10B is a diagram showing a result in the example 2;
FIG. 10C is a diagram showing a result in the example 2;
FIG. 10D is a diagram showing a result in the example 2;
FIG. 10E is a diagram showing a result in the example 2;
FIG. 10F is a diagram showing a result in the example 2;
FIG. 10G is a diagram showing a result in the example 2;
FIG. 10H is a diagram showing a result in the example 2;
FIG. 10I is a diagram showing a result in the example 2;
FIG. 11A is a diagram showing a result in an example 3;
FIG. 11B is a diagram showing a result in the example 3;
FIG. 11C is a diagram showing a result in the example 3;
FIG. 11D is a diagram showing a result in the example 3;
FIG. 11E is a diagram showing a result in the example 3;
FIG. 11F is a diagram showing a result in the example 3;
FIG. 11G is a diagram showing a result in the example 3;
FIG. 11H is a diagram showing a result in the example 3;
FIG. 11I is a diagram showing a result in the example 3;
FIG. 11J is a diagram showing a result in the example 3;
FIG. 11K is a diagram showing a result in the example 3;
FIG. 11L is a diagram showing a result in the example 3;
FIG. 11M is a diagram showing a result in the example 3;
FIG. 11N is a diagram showing a result in the example 3;
FIG. 11O is a diagram showing a result in the example 3; and
FIG. 11P is a diagram showing a result in the example 3.

### PREFERRED MODE FOR CARRYING OUT THE INVENTION

The present disclosure will be described below. It should be understood that a singular expression encompasses the concept of a plural unless otherwise stated, throughout the present specification. Therefore, it should be noted that an article of a singular (for example, "a", "an" or "the" in the case of English) encompasses the concept of a plural form thereof unless otherwise stated. Further, it should be noted that terms used in the present specification are used in meanings that are commonly used in the field unless otherwise stated. Therefore, all the technical terms and scientific terms used in the present specification have the same meanings as generally understood by those skilled in the art in the field to which the present disclosure belongs unless otherwise defined. In the case of contradiction, the present specification (including definitions) takes precedence.

### (Definitions)

First, terms and general technologies used in the present disclosure will be described.

In the present specification, "a reaction of a living body" refers to an arbitrary phenomenon that occurs in response to a stimulus given to the living body. As reactions of a living body, senses that the living body can recognize, such as the sense of pain, the sense of taste, the sense of sight, the sense of smell or the sense of hearing, and given. These senses can be expressed as physiological assessment items, and signals thereof can be referred to as biosignals.

In the present specification, a "model" and a "hypothesis" are synonymously used, and are expressed using a mathematical function or logical expression, with a mapping that describes a correspondence between an inputted prediction target object and a prediction result or with a set of candidates for the mapping. In learning in machine learning, training data is referred to, and a model thought to approximate a true model best is selected from a set of models.

As a model, a generation model, an identification model, a function model or the like is given. The above models are different in the policy of expressing a classification model of a mapping relationship between an input (a prediction target object) x and an output (a prediction result) y. The generation model expresses conditional distribution of the output y when the input x is given. The identification model expresses joint distribution of the input x and the output y. In the identification model and the generation model, the mapping relationship is stochastic. The function model is such that the mapping relationship is deterministic, and a deterministic function relationship between the input x and the output y is expressed. Though it may be said that the identification model is slightly higher in the accuracy than the generation model, there is basically no superiority or inferiority between the models according to the no-free-lunch theorem.

In the present specification, "machine learning" refers to a technology of giving an ability of learning to a computer without expressly performing programming. The machine learning is a process of a functional unit improving its own performance by acquiring new knowledge/technical skills or reconfiguring existing knowledge/technical skills. By programming a computer so as to learn from experience, most of time and effort required for programming details can be reduced. In the machine learning field, a method for constructing such a computer program that can automatically make improvements based on experience is discussed. Data analysis/machine learning are essential technologies to be the basis of intellectual processing alongside the algorithm field, and are generally used in conjunction with other technologies. Knowledge of the fields in conjunction (domain specific knowledge; for example, the medical field) is required. The range of application of data analysis/machine learning includes prediction (collecting data and predicting what is going to happen), search (finding some outstanding feature from among the collected data), verification/description (examining relationships among various elements in the data) and the like. Machine learning is based on an index indicating the degree of achievement of a goal in the real world, and a user of machine learning has to grasp the goal in the real world. It is necessary to formulate such an index that becomes better when the goal is achieved. Machine learning is an inverse problem and is such an ill-posed problem that whether its solution has been obtained or not is unknown. The behavior of a learned rule is not deterministic but probabilistic. Operational ingenuity on the assumption that some part that cannot be controlled remains is required. It is useful for the user of machine learning to sequentially select data and information according to the goal in the real world, referring to performance indexes for training time and operation time.

As machine learning, linear regression, logistic regression, support vector machine or the like can be used, and the discrimination accuracy of each model can be calculated by performing cross-validation (CV) (also referred to as cross-verification or cross-confirmation).

By, after performing ranking, increasing the number of features one by one to perform machine learning (linear regression, logistic regression, support vector machine or the like) and cross-verification, the discrimination accuracy of each model can be calculated. Thereby, a model with the highest accuracy can be selected. In the present disclosure, arbitrary machine learning can be used, and linear regression, logistic regression, support vector machine (SVM) or the like can be utilized as supervised machine learning.

In machine learning, logical reasoning is performed. There are roughly three kinds of logical reasoning, that is, deduction, induction, abduction and analogy. The deduction is such that, when there is a hypothesis that Socrates is a human, and all humans die, derives a conclusion that Socrates dies, which can be said to be a special conclusion. The induction is such that, when there is a hypothesis that Socrates dies, and Socrates is a human, derives a conclusion that all humans die. The induction derives a general rule. The abduction is such that, when there is a hypothesis that Socrates dies, and all humans die, derives a conclusion that Socrates is a human. The abduction corresponds to a hypothesis/description. In the induction, however, how generalization is to be performed depends on an assumption. Therefore, it should be noted that there is a possibility that the induction cannot be said to be objective. The analogy is such a probabilistic way of logical thinking that it is reasoned that, if there are objects A and B, the object A has four features, and the object B has three of the features in common, then the object B similarly has the remaining one feature, and the objects A and B have the same kind of or similar relatedness.

In machine learning, a feature/attribute indicates what state a prediction target object is in when the prediction target object is seen from a certain aspect. A feature vector/attribute vector is such that features (attributes) describing the prediction target object are collected in a vector format.

In the present specification, "reaction data" refers to data of a phenomenon that occurs in response to a stimulus to an object. In a case where the object is a living thing, the reaction data is data showing physiological activity, for example, the sense of pain of the targeted living thing. The reaction data includes, for example, brain wave data. In the present specification, the reaction data can be used synonymously with a biosignal.

In the present specification, a "stimulus" refers to such that causes some reaction to occur on an object. In the case where the object is a living thing, the stimulus is a factor that brings a temporary change in the physiological activity of the living thing or a part of the living thing.

### (Biosignal-related)

In the present specification, the term "object" is used synonymously with a patient or a subject, and refers to an arbitrary living body or an animal targeted by the technologies of the present disclosure such as pain measurement and brain wave measurement. The object is preferably a human but is not limited thereto. In the present specification, in the case of estimating a pain, an ache or a subjective assessment, an "estimation target object" may be used, which means the same meaning of a target or the like. In the present specification, an object from which a biosignal used at the time of creating a feature template or a model used for estimation of a pain, an ache or a subjective assessment is derived is referred to as a "modeling target object". A plurality of "objects" can exist. In such a case, each individual example may be referred to as a "sample" (of a target).

In the present disclosure, a biosignal refers to a signal obtained from a living body. The biosignal encompasses, for example, a brainwave signal showing the activity of the brain of the living body, an electromyogram signal showing the activity of muscles of the living body, an electrocardiographic signal showing the activity of the heart of the living body, a nerve signal communicated through nerve cells, a mechanomyographic signal showing the activity of muscles of the living body, a muscle hardness signal showing the hardness of muscles of the living body and the like but is not limited thereto. The biosignal is typically a brain wave signal. By performing a publicly known feature extraction process, feature data can be extracted from the biosignal. The feature data encompasses an amplitude, frequency power, coherence and entropy but is not limited thereto.

### (Brain-wave-related)

In the present specification, a "brain wave" is synonymous with the term that is commonly used in the brain wave field and refers to a current generated by a potential difference accompanying the neural activity of the brain and measured using a pair of electrodes placed on the scalp. The brain wave encompasses an electroencephalogram (EEG) in which temporal change in a current is derived and recorded. It is said that, during rest, a wave with an amplitude of about 50 uV and a frequency of about 10 Hz constitutes a main component. This is referred to as an α wave. It is said that, during mental activity, the α wave is suppressed, and a fast wave of 17 to 30 Hz with a small amplitude appears. This is referred to as a β wave. It is said that, during light sleep, the α wave gradually decreases, and a θ wave of 4 to 8 Hz appears. It is said that, during deep sleep, a δ wave of 1 to 4 Hz appears. Each of these brain waves can be expressed with a particular amplitude, frequency, complicatedness index, correlation and the like, and, in the present disclosure, can be indicated by a particular amplitude and frequency or by analysis of the amplitude.

In the present specification, the "brain wave data" is arbitrary data related to brain waves (also referred to as "brain activation", "brain features" or the like) and includes amplitude data (an EEG amplitude), frequency characteristics and the like. Since "analysis data" obtained by analyzing the above brain wave data can be used similarly to the brain wave data, the analysis data and the brain wave data may be collectively called "brain wave data or analysis data thereof" in the present specification. As the analysis data, for example, mean amplitudes and peak amplitudes (for example, Fz, Cz, C3 and C4) of brain wave data, frequency powers (for example, Fz(δ), Fz(θ), Fz(α), Fz(β), Fz(γ), Cz(δ), Cz(θ), Cz(α), Cz(β), Cz(γ), C3(δ), C3(θ), C3(α), C3(β), C3(γ), C4(5), C4(θ), C4(α), C4(β), C4(γ) and the like) and the like can be given. Of course, other data that is commonly used is not excluded, as the brain wave data or analysis data thereof. For example, if what is obtained only by cutting out raw data for a predetermined time is used for discrimination, it can be used in the present disclosure because it is a feature.

In the present specification, "brain wave features" or "features of a brain wave" refers to arbitrary features of a brain wave and encompasses the "brain wave data or analysis data thereof. For example, amplitudes, mutual relationships among brain wave features, frequency powers and complicatedness indexes and the like can be encompassed. As examples of the above, the amplitudes include mean amplitudes (for example, an absolute mean amplitude, a relative mean amplitude and the like) and amplitude distribution characteristic values such as a median amplitude, an amplitude mode, a large amplitude, a peak amplitude and a quartile amplitude; the mutual relationships among brain wave features include potential correlations (for example, frontal-vertex potential correlation (a correlation coefficient, a partial correlation coefficient, connectivity, causality and their subspecies)) or inter-electrode phase synchronization (for example, coherence, a phaselocking value and their subspecies); the frequency powers includes a spectral density, a power spectrum and their subspecies; and the complicatedness indexes can include at least one selected from entropies (for example, a multi-scale entropy (MSE), a sample entropy, a self-entropy, a mean entropy, a joint entropy, a relative entropy and a conditional entropy) and biopotential features that occur in conjunction with occurrence of a pain, related to the phenomenon (such as an eye movement potential reflecting an eye movement such as blink reflex).

In the present specification, the "amplitude data" is a kind of "brain wave data" and refers to data of the amplitude of a brain wave. The amplitude data may be referred to simply as an "amplitude" or an "EEC amplitude". Such amplitude data may be also referred to as "brain activity data", "a brain activity amount" or the like because it is an indicator of brain activity. The amplitude data can be obtained by measuring an electrical signal of a brain wave and is displayed as a potential (that can be displayed with a unit of uV or the like). As the amplitude data, a mean amplitude can be used, but the amplitude data is not limited thereto.

In the present specification, an "ache" and a "pain" are synonymous and refer to a sensation which, when there is a strong invasion to a body, such as an injury or an inflammation, occurs in response to the invasion as a stimulus. A pain is not a disease but a symptom, and the mode of the pain is determined by a combination of three main characteristics of central, nociceptive and neuropathic pains. An acute pain and a chronic pain are distinguished. These are different in the related a brain region network (connectivity). In the case of the chronic pain, a subjective report of being painful may be made in spite of actually not being painful, and such a psychogenic factor that cannot be explained based on the intensity of sensation to a painful stimulus is also included.

In the case of a human, as sensations accompanied by a strong uncomfortable feeling such as the sense of pain, general sensations are also included. In addition, a cutaneous pain and the like have the nature of external acceptance to some extent and are considered to be useful for judgment of the quality of a foreign matter, such as hardness, sharpness, heat (heat pain), coldness (cold pain) and hot taste, in cooperation with other skin sensations and the sense of taste. In addition to the skin and mucous membranes, the human sense of pain can occur at almost all parts of the body (for example, pleuras, peritonea, internal organs (visceral pain; except the brain), teeth, eyes and ears), and, at all the parts, the sense of pain can be perceived as a brain wave or fluctuations thereof in the brain. In addition, the sense of internal pain represented by visceral pain is also included as the sense of pain. The senses of pain described above are referred to as somatic pain in contrast to the visceral pain. In addition to the somatic pain and the visceral pain, the sense of pain called "referred pain", which is a phenomenon of having a pain on the surface of a site different from a site that is actually injured, is also reported.

Individual senses of pain are different in the susceptibility (a pain threshold). There is a qualitative difference due to a difference in how a pain stimulus occurs or in the receptor site, and there are classifications such as dull pain and sharp pain. In the present disclosure, however, any kind of sense of pain can be measured, estimated and classified. Further, the present disclosure can respond to the sense of fast pain (A sense of pain) and the sense of slow pain (B sense of pain), (fast) local pain and (slow) diffuse pain. The present disclosure can respond to an abnormal sense of pain such as abnormal hyperalgesia. As peripheral nerves that transmit a pain, two nerve fibers of an "Aδ fiber" and a "C fiber" are known. For example, when hands are clapped, a pain in the beginning is transmitted as a sharp pain, which is clearly localized (a primary pain; a sharp pain), by transmission by the Aδ fiber. It is said that, after that, a throbbing pain that is not clearly localized (a secondary pain; a dull pain) is felt by transmission by the C fiber. Pains are classified into "acute pain" that continues for four to six weeks or less and "chronic pain" that continues for four to six weeks or more. Though a pain is an important vital sign similar to pulsation, temperature, blood pressure and breathing, it is difficult to display it as objective data. The VAS (visual analogue scale) and the Faces Pain Rating Scale, which are representative pain scales, are subjective assessment method and are unable to compare pains experienced by two or more patients. On the other hand, the present inventor paid attention to brain waves that are less susceptible to influence of the peripheral circulatory system, as an indicator for objective assessment of a pain, and it was derived that, by observing changes in the amplitude/latency of the brain waves in response to a pain stimulus and performing trend analysis, discrimination and classification of a pain are possible. It is possible to detect an instant stimulus and a continuous stimulus. Especially, as for an instant pain and a continuous throbbing pain, there is a possibility that such pains can be distinguished by the analysis of the present disclosure. Since the instant pain is a pain during a short time section, there is a possibility that, when a temporal direction averaging for at least tens of seconds is used, related brain activity attenuates (example: a significant correlation with pain assessment is not seen). On the other hand, in the case of the continuous pain, there is a possibility that the significant correlation with pain assessment is, on the contrary, strengthened by the temporal direction averaging because the continuous pain lasts. Further, the present inventor also paid attention to the brain waves that are less susceptible to influence of the peripheral circulatory system as an index for objective assessment of a pain, and it was found that, by observing changes in the amplitude/latency of the brain waves in response to a pain stimulus and applying a reference stimulus, the accuracy thereof increases.

In the present disclosure, it is one of important points that not the intensity itself but whether the pain is a pain "that requires treatment" or not can be distinguished, and it is possible to diagnose it more accurately by the reference stimulus. Therefore, it is also important that "pains" can be clearly categorized based on the concept of "treatment". For example, it can be said that "quantitative" classification of pains, such as "comfortable/uncomfortable" and "unbearable" pains, can be led. For example, positioning of a "pain index", a baseline and a relationship therebetween can be defined, and it is assumed that the case of n=3 or more is also possible in addition to the case of n=2. In the case of three or more, classification into "not painful", "comfortably painful" and "painful" can be performed. For example, discrimination among a pain that is "unendurable and requiring treatment", "middle" and "painful but not bothering" can be made. In the case of using the analysis of the present disclosure, it is possible to, by identifying a threshold for the length of the duration of a signal related to a strong pain, distinguish "unendurable" and "painful but endurable" pains.

In the present specification, a "subjective pain sensation level" refers to a level of the sensation of pain that an object has, and can be expressed by a conventional technology such as a computerized visual analogue scale (COVAS) or other publicly known technologies, for example, Support Team Assessment Schedule (STAS-J), Numerical Rating Scale (NRS), Faces Pain Scale (FPS), Abbey pain scale (Abbey), Checkinlist of Nonverbal Pain Indicatiors (CNPI), Non-communicative Patient's Pain Assessment Instrument (NOPPAIN) and Doloplus 2.

The "information criterion" used in the present disclosure is representatively BIC (the Bayesian information criterion), but other information criteria can be also used. The other information criteria can include the following but are not limited thereto.
- AIC (Akaike Information Criterion)
- MDL (Minimum Description Length) Criterion
- ABIC (Akaike Bayesian Information Criterion)
- CIC (Contrast based Information Criterion)
- DIC (Deviance Information Criterion)
- EIC (Bootstrap Information Criterion: Empirical Information Criterion)
- GIC (Generalized Information Criterion)
- PIC (Predictive Information Criterion)
- TIC (Takeuchi's Information Criterion)
- WAIC (Widely Applicable Information Criterion)
- WBIC (Widely applicable Bayesian information criterion)

### (Concept of algorithm of the present disclosure)

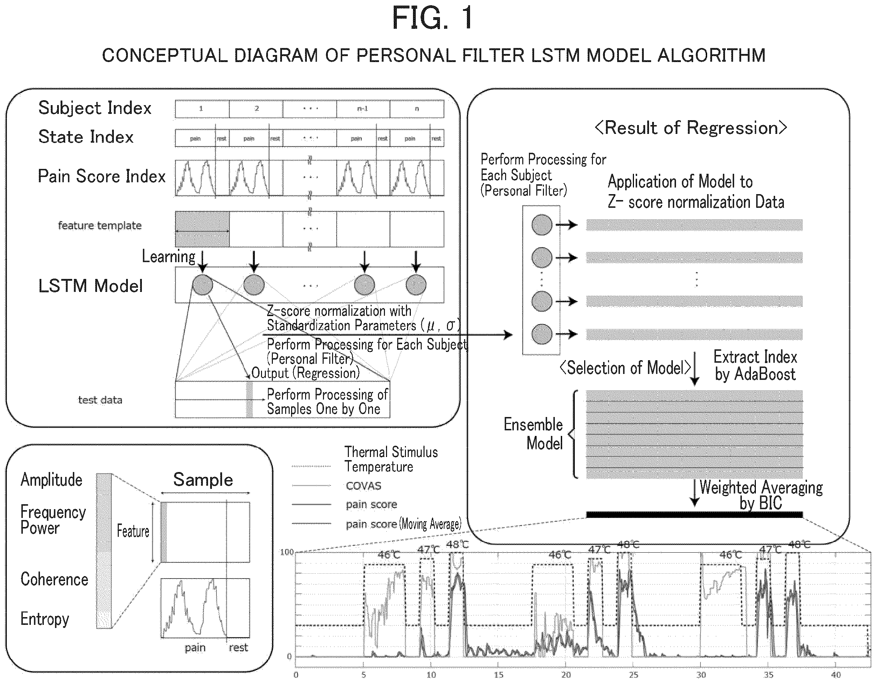

FIG. 1 is a diagram showing the concept of an example of an algorithm of the present disclosure. FIG. 1 shows a conceptual diagram of an LSTM (long short-term memory) model algorithm using an LSTM model.

In the LSTM model algorithm, n LSTM models are created using n biosignals obtained from n modeling target objects, respectively, and a pain experienced by an object can be estimated using the n LSTM models.

Each LSTM model has such a three-dimensional data structure of "n samples×1 sequence×m features". For example, an LSTM can be a bi-directional LSTM using a simple one-layer network. In the present example, a network structure and parameters as shown below can be used.
1. 'sequenceinput': sequence input; 324-dimensional sequence input
2. 'biLSTM': BiLSTM; BiLSTM with 300 hidden units
3. "dropout": dropout; 50% dropout
4. 'fc': full connection; one full connection layer
5. 'regressionoutput': regression output; mean-squared-error of response 'Response'
Hidden units: 300
Dropout: 0.5
Solver for learning of network: Adam optimizer
Initial learning rate: 0.001
Attenuation rate of moving average of gradient squared: 0.99
Mini-batch size: 128
Number of training epochs: 50
L2 regularization: 0.01

The above hyper parameters can be used being fixed, without performing cross validation.

For example, an index indicating a pain that an estimation target object feels, a pain score can be estimated by the following procedure.
1. Perform extraction of features of test data (perform the extraction of features in the same method as when LSTM models were created in advance (the procedure for the extraction of features and parameters necessarily need to correspond)). Here, the test data refers to data obtained from the object targeted by estimation of a pain. The test data can mean both of test data in a broad sense that means brain wave data that has not been pre-processed at all and test data in a narrow sense that means data obtained by extracting features from the brain wave data and performing a process for standardizing the features with standardization parameters.
2. An LSTM model (a model obtained by combining a plurality of models) created in advance is loaded. Here, the "model obtained by combining a plurality of models" means such that is obtained by combining models created for modeling target objects, respectively.
3. For example, it is assumed that the model loaded at 2 is configured with four champion models (a, b, c and d) that were confirmed to have a high application accuracy in advance among about ninety models. Specifically, for each of the four champion models, twenty standardizations (z-score normalization) using twenty kinds of standardization parameters (average values and standard deviations) are performed in advance for the test data from which the features have been extracted. For example, for a model a, twenty pieces of standardized data, that is, a-param (1), a-param (2), ..., a-param (20) can be defined, and application by the model a is performed for each of them. For the data of a-param (n), it is necessary to perform application by the model a. When this is repeated similarly for the models b, c and d, it becomes possible to express 4 (the number of models)×20 (the number of standardization parameters)=80 pain scores.
4. Which result (pain score) is to be adopted from among the eighty pain scores is selected. As a method for the selection, various kinds of methods can be used. For example, the following method can be adopted. The selection of a pain score is the most important process that influences a result. As the first stage, by selecting one, more or all optimal pain scores are selected from the twenty standardization parameters in one model and determining an ensemble average thereof to make them one. At the second stage, an ensemble average of the ensemble averages is determined. That is, at the first stage, for each of the four champion models, one, more or all optimal pain scores are selected, and an ensemble average thereof is determined to make them one.
5. As a method for determining the ensemble average, an average value of pain scores may be simply determined, or a weighted average of pain scores may be determined using BIC (Bayesian information criterion). An arbitrary information criterion can be used instead of the BIC. As for selection of pain scores, a method of extracting such pain score indexes that the error is below 0.5 using AdaBoost and selecting the indexes may be used.
6. Further, selection may be performed by the following method. For example, as a reference of selection of pain scores, a pain score during rest or when a task of intentionally causing noise to occur (a noise session) is imposed on an estimation target object is assumed to be zero indicating that pain has not occurred at all. Then, by selecting such combinations of model and standardization parameter that minimize occurrence of a false positive, which is an error of judging that pain has occurred though it has not occurred, it becomes possible to express highly accurate pain scores.

In the above example, it has been described that n LSTM models are created using n biosignals obtained from n modeling target objects, respectively, and a pain experienced by an estimation target object is estimated using the n LSTM models. However, the number of modeling target objects and the number of created models do not have to be the same. For example, a number of LSTM models smaller than n may be created using n biosignals obtained from n modeling target objects, respectively. For example, biosignals of m modeling target objects with similar brain waves may be mixed to create one model. Further, the number of created models and the number of models actually used do not have to be the same. For example, in a case where n models are created, a pain experienced by an estimation target object may be estimated using a number of models smaller than n.

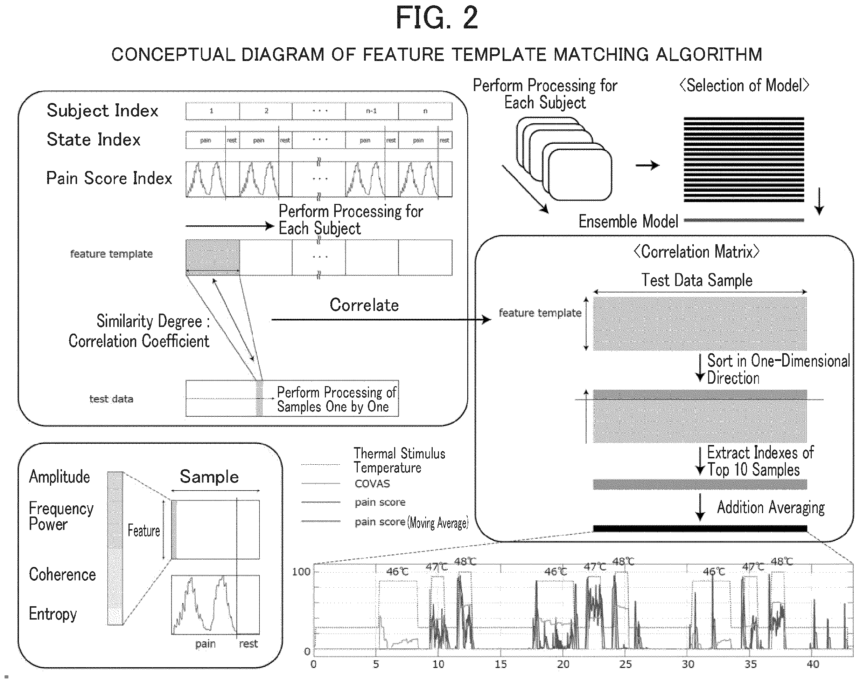

FIG. 2 is a diagram showing the concept of another example of the algorithm of the present disclosure. FIG. 2 shows a conceptual diagram of a feature template matching algorithm that uses feature templates.

In the feature template matching algorithm, n feature templates are created using n biosignals obtained from n modeling target objects, respectively, and a pain experienced by an object can be estimated using the n feature templates.

A feature template refers to such that features extracted from brain wave data correspond to pain labels, respectively, one-to-one.

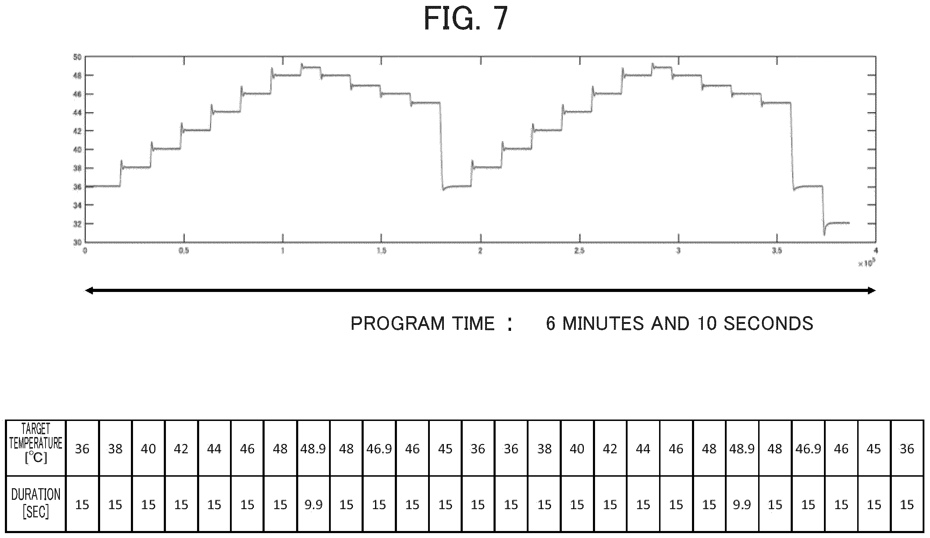

Each feature template can be created, for example, by the following procedure:
1. By giving each modeling target object such a heat stimulus that temperature rises from 36°C to 49°C and drops in steps twice (see FIG. 7) in advance, obtained brain wave data of pain and labels (a trigger and a COVAS template) are read. The COVAS template can be created by, after experiments of the n modeling target objects and before creation of the feature templates, performing addition averaging of pieces of data corresponding to the n modeling target objects and fitting the scale to 0 to 100.
2. First, as preprocessing, a total of 18-ch time-series brain wave data, that is, 6-ch brain wave data (monopolar) of the forehead, 6-ch bipolar data derived using the 6-ch brain wave data and a 6-ch CAR (common average reference) is extracted.
3. After applying noise processing using a trapezoidal filter, N samples are cutout by shifting a 16-second window (a large window) by one second each time for the total length of each piece of time-series brain wave data. The size is 16000 samples×N when the sampling frequency is assumed to be 1000 Hz. A similar process is also performed for the COVAS template labels. Each new COVAS label is defined by an average value of the cut-out window size (16000 samples) so that the new COVAS labels correspond to N brain wave samples, respectively, one-to-one. At this time, the size of the COVAS labels is 1×N.
4. Inside each cut-out large window, nine different sequences to be time information are defined by further shifting an 8-second window (a small window) by one second each time. It is because LSTM is strong in processing in consideration of time information that the sequences are defined.
5. For one small window, a process for extracting the following four kinds of features (amplitude, frequency power, coherence and entropy) is performed. For the frequency power and the coherence, eight frequency bands are defined, that is, δ(2-5 Hz), θ(5-8 Hz), α(8-14 Hz), β(14-28 Hz), γ(28-58 Hz), γ2(62-118 Hz), γ3(122-178 Hz) and γ4(182-238 Hz).
   (1) Amplitude: 18 amplitudes are extracted for 18 ch.
   (2) Frequency power: 144 frequency powers of 8 frequency bands are extracted for 18 ch.
   (3) Coherence: 144 coherences of 8 frequency bands are extracted for 18 ch.
   (4) Entropy: 18 entropies are extracted for 18 ch. A total of 324 features can be extracted.
6. At this point of time, three-dimensional data of brain wave features N×9×324, and 1×N COVAS labels have been extracted. The three-dimensional data has a data structure used at the time of creating the LSTM model described above. At the time of performing feature template matching for test data, the nine pieces of sequence information are not necessary. Therefore, only the first sequence is used. The data structure at that time is two-dimensional data of N×324.
7. As experiment data, data during rest (rest) and data at a time when a heat stimulus is given (heat) exist. It is known that data obtained by combining both data in an appropriate proportion is effective as learning data. For example, by using a COVAS label as a heat label and using such a label that all the values are 0 as a rest label, it becomes possible to express strength/weakness of pain including the pain during rest.
8. A feature template for one person is defined by combining rest and heat data. What is obtained by combining pieces of data from which features have been extracted, for n persons is an overall large feature template, in which labels corresponding to the pieces of data from which features have been extracted, respectively, one-to-one are also completely included.

For example, a pain score, which is an index indicating a pain that an estimation target object feels, can be estimated by the following procedure.
1. Perform extraction of features of test data (perform the extraction of features in the same method as when feature templates were created in advance (the procedure for the extraction of features and parameters necessarily need to correspond)). Here, the test data refers to data obtained from the object targeted by estimation of a pain. The test data can mean both of test data in a broad sense that means brain wave data that has not been pre-processed at all and test data in a narrow sense that means data obtained by extracting features from the brain wave data and performing a process for standardizing the features with standardization parameters.
2. A feature template (a feature template obtained by combining a plurality of feature templates) created in advance is loaded. Here, the "feature template obtained by combining a plurality of feature templates" means such that is obtained by combining feature templates created for modeling target objects, respectively.
3. For example, it is assumed that the model loaded at 2 is configured with four champion feature templates (a, b, c and d) that were confirmed to have a high application accuracy in advance among about ninety feature templates.
4. For example, when a feature template a is assumed to be configured with n samples×m features, the m features are standardized for all the n samples of the feature template a, and test data is standardized with quite the same standardization parameters (m average values and standard deviations) at the time of performing application.
5. When correlation coefficients are determined between all the n samples of the standardized feature template and the standardized test data, n correlation coefficients are obtained for test data at certain time. A label (here, a COVAS average value is used) that a sample with the highest correlation coefficient among the n correlation coefficients has can be defined as a pain score at the current point of time. Here, a feature template and a label are defined in a one-to-one correspondence. When one sample of a feature template is selected, one corresponding label is also selected.
6. The accuracy can be higher by determining an ensemble average of a plurality of samples than by selecting one sample with the highest correlation coefficient. For example, the samples may be sorted in descending order of the correlation coefficients, and an ensemble average of top ten samples may be taken. The number of samples the ensemble average of which is to be taken may be any number, for example, 100 or 200. In this way, a pain score at the first stage is determined.
7. By similarly processing the four champion feature templates by the method as described above and using ensemble averages of scores for the champion feature templates, a pain score of the second stage is determined. The pain score may be set as a pain score outputted in the end.

In the above example, it has been described that n feature templates are created using n biosignals obtained from n modeling target objects, respectively, and a pain experienced by an estimation target object is estimated using the n feature templates. However, the number of modeling target objects and the number of created feature templates do not have to be the same. For example, a number of feature templates smaller than n may be created using n biosignals obtained from n modeling target objects, respectively. For example, biosignals of m modeling target objects with similar brain waves may be mixed to create one feature template. Further, the number of created feature templates and the number of feature templates actually used do not have to be the same. For example, in a case where n feature templates are created, a pain experienced by an estimation target object may be estimated using a number of feature templates smaller than n.

### (Preferred embodiments)

Preferred embodiments of the present disclosure will be described below. It is understood that the embodiments provided below are provided for better understanding of the present disclosure, and the scope of the present disclosure should not be limited to the description below. Therefore, it is clear that one skilled in the art can appropriately make alterations within the scope of the present disclosure by referring to the description in the present specification. Further, it is understood that each of the embodiments of the present disclosure below can be used alone, or the embodiments can be combined and used.

All of the embodiments described below show comprehensive or specific examples. Numerical values, shapes, materials, components, arrangement positions and connection forms of the components, steps, orders of steps and the like shown in the embodiments below are mere examples and are not intended to limit the claims. Further, among the components in the embodiments below, components that are not described in independent claims indicating top concepts will be described as arbitrary components.

In one aspect, the present disclosure provides a system 100 for estimating a subjective assessment made by an estimation target object. The system 100 for estimating a subjective assessment made by an estimation target object is configured to, using feature data of a biosignal acquired from the estimation target object when a predetermined phenomenon occurs, estimate a subjective assessment of the predetermined phenomenon made by an estimation target object.

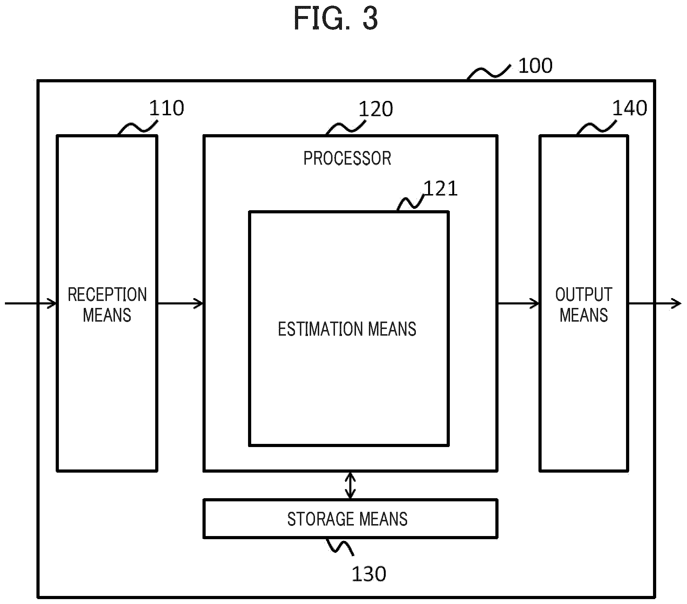

FIG. 3 is a diagram showing an example of the configuration of the system 100 for estimating a subjective assessment made by an estimation target object.

The system 100 is provided with a reception means 110, a processor 120, a storage means 130 and an output means 140.

The reception means 110 is configured to receive feature data of a biosignal acquired form an estimation target object. It does not matter from where the reception means 110 receives the feature data. The reception means 110 may be adapted to receive the feature data from outside the system 100 or from inside the system 100. For example, the system 100 may be adapted to receive a biosignal from outside the system 100 and receive feature data extracted from the biosignal using an extraction means that the system 100 can be provided with. The extraction means that extracts a biosignal may be implemented with the processor 120 or by other means. In the case where the extraction means that extracts a biosignal is implemented with the processor 120, the reception means 110 only has to receive a biosignal acquired from an estimation target object. The reception means 110 can hand over the received biosignal to the processor 120, and the processor 120 can perform subsequent processing after extracting feature data from the received biosignal.

For example, the reception means 110 may be adapted to receive feature data of a biosignal when a load is given to an estimation target object or receive no-load feature data of a biosignal when a load is not given to an estimation target object. The no-load feature data can be used, for example, to select at least one of a plurality of feature templates to be used to estimate a subjective assessment made by an estimation target object. The no-load feature data can be used, for example, to select at least one of a plurality of models to be used to estimate a subjective assessment made by an estimation target object.

The reception means 110 can hand over received feature data to the processor 120, and the processor 120 can receive the feature data.

The processor 120 executes processes of the system 100 and controls the whole operation of the system 100. The processor 120 reads out a program stored in the storage means 130 (a memory) and executes the program. Thereby, it is possible to cause the system 100 to function as a system that executes desired steps. The processor 120 may be adapted to, if feature data received by the reception means 110 is in a format not suitable for processing, perform a process for converting the format to a format suitable for processing. The processor 120 may be implemented with a single processor or with a plurality of processors.

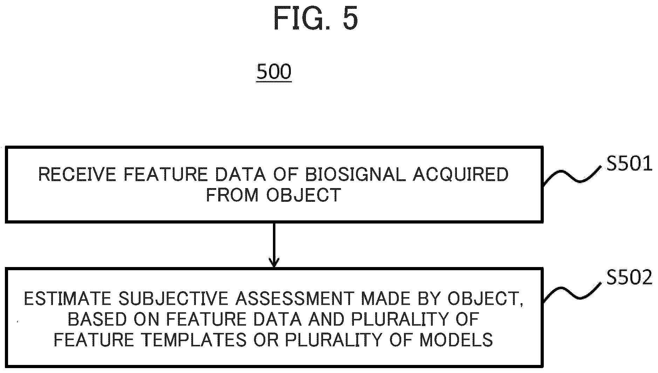

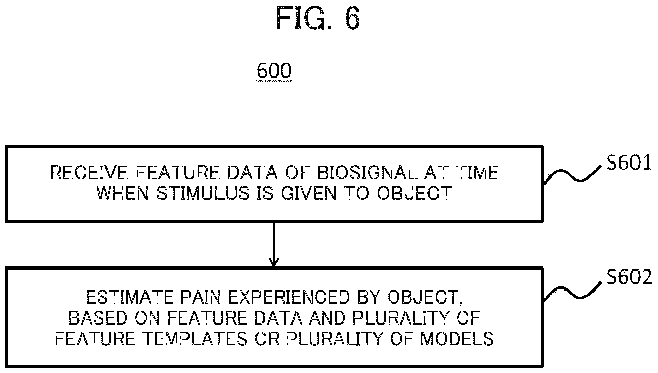

The storage means 130 includes a memory. The memory stores programs required to execute the processes of the system 100 and data and the like required to execute the program. The memory may store a program for causing the processor 120 to perform a process for estimating a subjective assessment made by an estimation target object (for example, a program that realizes processes shown in FIGS. 5 and 6 to be described later). The processor 120 may store a program for causing the processor 120 to perform a process for identifying a reaction of a living body using a constructed model. The processor 120 may store a program that realizes a process for extracting feature data from a biosignal. Here, it does not matter how the programs are stored into the memory. For example, the programs may be pre-installed in the memory. Or alternatively, the programs may be installed into the memory by being downloaded via a network. In this case, the type of the network does not matter.

The storage means 130 includes a database. The database stores a plurality of feature templates extracted from a plurality of biosignals or a plurality of models that have learned the feature templates.

A feature template refers to a template in which features extracted from brain wave data correspond to pain labels, respectively, one-to-one. For example, in a feature template, pieces of feature data of a plurality of samples sampled from a biosignal are associated with values indicating subjective assessments, respectively. A feature template can be created from each of a plurality of biosignals acquired from a plurality of modeling target objects. The plurality of modeling target objects include a first modeling target object, a second modeling target object, ..., and an n-th modeling target object; and the feature templates include a first feature template extracted from a first biosignal acquired from the first modeling target object, a second feature template extracted from a second biosignal acquired from the second modeling target object, ..., and an n-th feature template extracted from an n-th biosignal acquired from the n-th modeling target object. Here, n is an integer equal to or larger than 2.

Each of the feature templates can be created, for example, by the following procedure:
1. Brain wave data of pain and labels (a trigger and a COVAS template) obtained by giving each modeling target object such a heat stimulus that temperature rises from 36°C to 49°C and drops in steps twice (see FIG. 7) in advance are read. The COVAS template can be created by, after experiments of the n modeling target objects and before creation of the feature templates, performing addition averaging of pieces of data corresponding to the n modeling target objects and fitting the scale to 0 to 100.
2. First, as preprocessing, a total of 18-ch time-series brain wave data, that is, 6-ch brain wave data (monopolar) of the forehead, 6-ch bipolar data derived using the 6-ch brain wave data and a 6-ch CAR (common average reference) is extracted.
3. After applying noise processing using a trapezoidal filter, N samples are cutout by shifting a 16-second window (a large window) by one second each time for the total length of each piece of time-series brain wave data. The size is 16000 samples×N when the sampling frequency is assumed to be 1000 Hz. A similar process is also performed for the COVAS template labels. Each new COVAS label is defined by an average value of the cut-out window size (16000 samples) so that the new COVAS labels correspond to N brain wave samples, respectively, one-to-one. At this time, the size of the COVAS labels is 1×N.
4. Inside each cut-out large window, nine different sequences to be time information are defined by further shifting an 8-second window (a small window) by one second each time. It is because LSTM is strong in processing in consideration of time information that the sequences are defined.
5. For one small window, a process for extracting the following four kinds of features (amplitude, frequency power, coherence and entropy) is performed. For the frequency power and the coherence, eight frequency bands are defined, that is, δ(2-5 Hz), θ(5-8 Hz), α(8-14 Hz), β(14-28 Hz), γ(28-58 Hz), γ2(62-118 Hz), γ3(122-178 Hz) and γ4(182-238 Hz).
   (1) Amplitude: 18 amplitudes are extracted for 18 ch.
   (2) Frequency power: 144 frequency powers of 8 frequency bands are extracted for 18 ch.
   (3) Coherence: 144 coherences of 8 frequency bands are extracted for 18 ch.
   (4) Entropy: 18 entropies are extracted for 18 ch. A total of 324 features can be extracted.
6. At this point of time, three-dimensional data of brain wave features N×9×324, and 1×N COVAS labels have been extracted. The three-dimensional data has a data structure used at the time of creating the LSTM model described above. At the time of performing feature template matching for test data, the nine pieces of sequence information are not necessary. Therefore, only the first sequence is used. The data structure at that time is two-dimensional data of N×324.
7. As experiment data, data during rest (rest) and data at a time when a heat stimulus is given (heat) exist. It is known that data obtained by combining both data in an appropriate proportion is effective as learning data. For example, by using a COVAS label as a heat label and using such a label that all the values are 0 as a rest label, it becomes possible to express strength/weakness of pain including the pain during rest.
8. A feature template for one person is defined by combining rest and heat data. What is obtained by combining pieces of data from which features have been extracted, for n persons is an overall large feature template, in which labels corresponding to the pieces of data from which features have been extracted, respectively, one-to-one are also completely included.

Each model is configured to output a value indicating a subjective assessment in response to an input of feature data. For example, each model can be configured to output a value indicating a pain (for example, a pain score) in response to an input of feature data.

A method used for learning for constructing each model can be an arbitrary method. The method used for learning can be, for example, LSTM (long-short-term memory). For example, by causing an LSTM to perform learning using three-dimensional data of brain wave features N×9×324 and 1×N COVAS labels, a model can be created. For example, in the case of constructing an LSTM model capable of predicting a pain, learning is performed by using a feature temperate as an input to an LSTM and using values of pain score indexes as labels (teacher outputs) thereof. In response to an input of feature data to the LSTM model constructed in this way, a pain score is outputted. The models can be created from the plurality of biosignals acquired from the plurality of modeling target objects. The plurality of modeling target objects include the first modeling target object, the second modeling target object, ..., and the n-th modeling target object; and the models include a first model that has learned the first feature template extracted from the first biosignal acquired from the first modeling target object, a second model that has learned the second feature template extracted from the second biosignal acquired from the second modeling target object, ..., and an n-th model that has learned the n-th feature template extracted from the n-th biosignal acquired from the n-th modeling target object. Here, n is an integer equal to or larger than 2.

Each LSTM model can have such a three-dimensional data structure that "n samples×1 seqeunce×m features" is obtained. For example, an LSTM can be a bi-directional LSTM using a simple one-layer network. In the present example, a network structure and parameters as shown below can be used.
1. 'sequenceinput': sequence input; 324-dimensional sequence input
2. 'biLSTM': BiLSTM; BiLSTM with 300 hidden units
3. "dropout": dropout; 50% dropout
4. 'fc': full connection; one full connection layer
5. 'regressionoutput': regression output; mean-squared-error of response 'Response'
Hidden units: 300
Dropout: 0.5
Solver for learning of network: Adam optimizer
Initial learning rate: 0.001
Attenuation rate of moving average of gradient squared: 0.99
Mini-batch size: 128
Number of training epochs: 50
L2 regularization: 0.01

The above hyper parameters can be used being fixed, without performing cross validation.

The database further stores a plurality of standardization parameters extracted from a plurality of biosignals. The plurality of standardization parameters include a plurality of first standardization parameters extracted from the first biosignal acquired from the first modeling target object, a plurality of second standardization parameters extracted from the second biosignal acquired from the second modeling target object, ..., and a plurality of n-th standardization parameters extracted from the n-th biosignal acquired from the n-th modeling target object. Thus, a plurality of standardization parameters can be extracted from one biosignal. In one example, twenty kinds of standardization parameters can be extracted from one biosignal. The standardization parameters include, for example, an average value µ and a standard deviation σ.

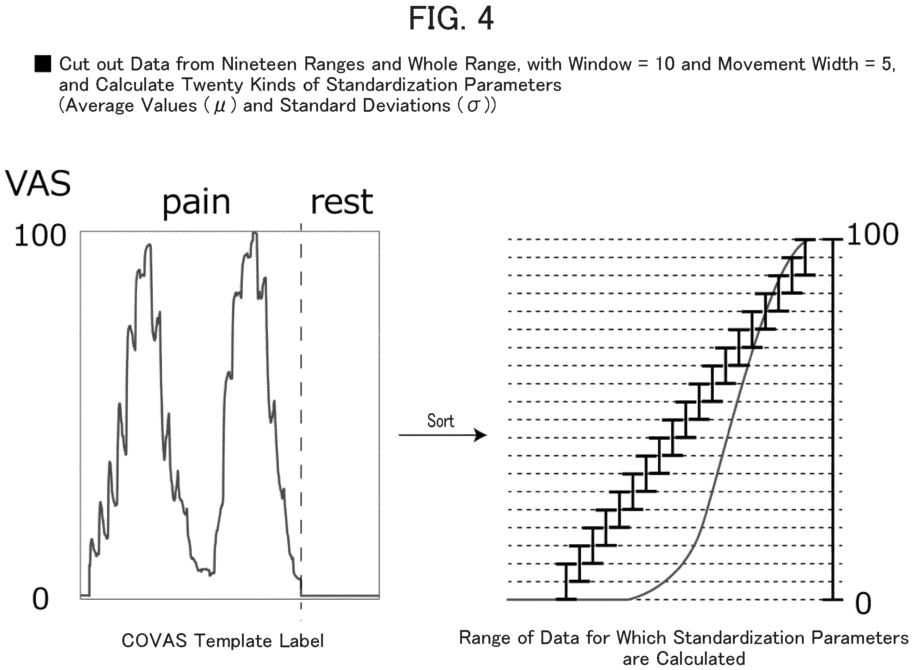

In one example, each standardization parameter is created by a method shown in FIG. 4. COVAS templates created in advance are sorted in ascending order from a minimum value 0 to a maximum value 100. From the sorted COVAS templates, pieces of data are cut out from nineteen ranges and the whole range from the minimum value 0 to the maximum value 100, with window=10 and movement width=5. An average value and a standard deviation of data of each of the twenty ranges are determined.

The output means 140 is configured to be capable of outputting data to the outside of the system 100. For example, the output means 140 can output data showing an estimated subjective assessment by an estimation target object. It does not matter in what form the output means 140 outputs data. For example, if the output means 140 is a display, the display may output data by displaying the data. For example, if the output means 140 is a transmitter, the transmitter may output data by transmitting the data to the outside of the system 100 via a network. For example, if the output means 140 is a writing device, the writing device may output data by writing the data to a storage medium or a database connected to the system 100. For example, the output means 140 may output data by converting the format to a format that can be handled by data output destination hardware or software or by adjusting the response speed to a response speed that can be handled by the data output destination hardware or software.

The processor 120 is provided with an estimation means 121.

In one embodiment, the estimation means 121 estimates a subjective assessment made by an estimation target object, based on feature data received by the reception means 110 and the plurality of feature templates stored in the storage means 130.

For example, the estimation means 121 can be configured to acquire a plurality of correlation sets by correlating the received feature data with each of the plurality of feature templates and estimate the subjective assessment by the estimation target object based on the plurality of correlation sets. Each of the feature templates includes feature data of each of a plurality of samples sampled from a biosignal of a modeling target object. By correlating one feature template with the received feature data, a correlation coefficient set having the same number of correlation coefficients as the number of samples can be obtained. For example, if a feature template has n samples, one correlation coefficient set has n correlation coefficients (for example, correlation coefficients within a range from -1 to 1) . A correlation coefficient set is acquired from each of the plurality of feature templates, and, as a result, a plurality of correlation coefficient sets are acquired.

The estimation means 121 can estimate the subjective assessment by the estimation target object using the plurality of correlation coefficient sets by the following procedure.

First, the estimation means 121 identifies, for each of the plurality of correlation coefficient sets, a value indicating a subjective assessment that is associated with a sample corresponding to the highest correlation coefficient. The value indicating a subjective assessment can be, for example, the value of a COVAS label associated with each sample. For example, the value of a COVAS label associated with a sample corresponding to the highest correlation coefficient in a first correlation coefficient set (a first COVA label value), the value of a COVAS label associated with a sample corresponding to the highest correlation coefficient in a second correlation coefficient set (a second COVA label value), ..., and the value of a COVAS label associated with a sample corresponding to the highest correlation coefficient in an n-th correlation coefficient set (an n-th COVA label value) are identified. Secondly, the estimation means 121 takes an ensemble average of the values indicating the subjective assessments of the plurality of correlation coefficient sets. For example, the estimation means 121 can take an ensemble average of the first COVA label value, the second COVA label value, ..., and the n-th COVA label value. Determining the ensemble average may be merely determining an average value of the COVA label values, or may be determining a weighted average of the COVA label values using a BIC (Bayesian information criterion). An arbitrary information criterion can be used instead of the BIC. Thirdly, the estimation means 121 determines a score (for example, a pain score) indicating the subjective assessment based on the ensemble average.

In another example, the estimation means 121 can estimate the subjective assessment by the estimation target object using the plurality of correlation coefficient sets by the following procedure.

First, the estimation means 121 identifies, for each of the plurality of correlation coefficient sets, values indicating subjective assessments that are associated with samples corresponding to a top plurality of correlation coefficients. The values indicating subjective assessments can be, for example, the values of COVAS labels associated with the samples. For example, the values of COVAS labels associated with samples corresponding to a top plurality of correlation coefficients in the first correlation coefficient set (a first COVA label value group), the values of COVAS labels associated with samples corresponding to a top plurality of correlation coefficients in the second correlation coefficient set (a second COVA label value group), ..., and the values of COVAS labels associated with samples corresponding to a top plurality of correlation coefficients in the n-th correlation coefficient set (an n-th COVA label value group) are identified. As for the samples of the top plurality of values indicating subjective assessments, such indexes of values of COVAS labels that the error is below 0.5 are extracted using AdaBoost, and the indexes may be selected. For example, the samples of the top plurality of values indicating subjective assessments can be selected so that, when a pain score during rest or at the time of imposing a task of intentionally causing noise to occur (a noise session) on an estimation target object is assumed to be 0 indicating that pain has not occurred at all, occurrence of a false positive, which is an error of judging that a pain has occurred though it has not occurred, is reduced as long as possible. Secondly, for each of the correlation coefficient sets, the estimation means 121 obtains an ensemble average correlation coefficient by taking an ensemble average of the values indicating the top plurality of subjective assessment. For example, the estimation means 121 can obtain a first ensemble average correlation coefficient by taking an ensemble average for the first COVA label value group, can obtain a second ensemble average correlation coefficient by taking an ensemble average for the second COVA label value group, ..., and can obtain an n-th ensemble average correlation coefficient by taking an ensemble average for the n-th COVA label value group. Determining the ensemble average may be merely determining an average value of the COVA label values, or may be determining a weighted average of the COVA label values using a BIC (Bayesian information criterion). An arbitrary information criterion can be used instead of the BIC. Thirdly, the estimation means 121 takes an ensemble average of the ensemble average correlation coefficients of the plurality of correlation coefficient sets. For example, the estimation means 121 can take an ensemble average of the first ensemble average correlation coefficient, the second ensemble average correlation coefficient, ..., and the n-th ensemble average correlation coefficient. Determining the ensemble average may be merely determining an average value of the ensemble average correlation coefficients, or may be determining a weighted average of the ensemble average correlation coefficients using a BIC (Bayesian information criterion). An arbitrary information criterion can be used instead of the BIC. Fourthly, the estimation means 121 determines a score (for example, a pain score) indicating the subjective assessment based on the ensemble average.

The estimation means 121 can use arbitrary a similarity degree in addition to the correlation coefficient. An index indicating a similarity degree encompasses, for example, a Bhattacharya distance, Bray and Curtis distance, Canberra distance, Chebychev distance, Chi2 distance, Chi2 metric, chord distance, squared chord distance, Euclidean distance, geodesic distance, Kendall dissimilarity, Mahalanobis distance, Manhattan distance, Ochiai's index, Pearson dissimilarity, Spearman dissimilarity, Chebyshev distance, Minkowski distance and cosine similarity but are not limited thereto.

In this way, the estimation means 121 can output data showing time-series change in the subjective assessment by the estimation target object. By interpreting the data, it is possible to understand what is the subjective assessment by the estimation target object.

At this time, the estimation means 121 need not give a load to the estimation target object in advance. Therefore, according to the system of the present disclosure, it is possible to highly accurately predict the subjective assessment by the estimation target object without giving a load to the estimation target object in advance. Since giving a load to the estimation target object is a burden on the estimation target object, it is useful that it is not necessary to give a load in advance.

In another embodiment, the estimation means 121 estimates a subjective assessment made by an estimation target object, based on feature data received by the reception means 110 and the plurality of models stored in the storage means 130.

For example, the estimation means 121 can be configured to acquire a plurality of outputs by inputting the received feature data to each of the plurality of models, and estimate the subjective assessment by the estimation target object based on the plurality of outputs. The plurality of outputs include a first output outputted from the first model, a second output outputted from the second model, ..., and an n-th output outputted from the n-th model. Here, n is an integer equal to or larger than 2.

The estimation means 121 can estimate the subjective assessment by the estimation target object using the plurality of outputs by the following procedure.

First, the estimation means 121 takes an ensemble average of the plurality of outputs. Determining the ensemble average may be merely determining an average value of the plurality of outputs, or may be determining a weighted average of the plurality of outputs using a BIC (Bayesian information criterion). An arbitrary information criterion can be used instead of the BIC. Secondly, the estimation means 121 determines a score (for example, a pain score) indicating the subjective assessment based on the ensemble average.

In this way, the estimation means 121 can output data showing time-series change in the subjective assessment by the estimation target object. By interpreting the data, it is possible to understand what is the subjective assessment by the estimation target object.

At this time, the estimation means 121 need not give a load to the estimation target object in advance. Therefore, according to the system of the present disclosure, it is possible to highly accurately predict a subjective assessment by an estimation target object without giving a load to the estimation target object in advance. Since giving a load to an estimation target object is a burden on the estimation target object, it is useful that it is not necessary to give a load in advance.

Z-score normalization is performed using each of a plurality of standardization parameters extracted from a plurality of biosignals. The plurality of standardization parameters are stored in the storage means 130.

The estimation means 121 generates a plurality of pieces of standardized feature data by performing standardization (z-score normalization) of the feature data received by the reception means 110. By performing standardization, it is possible to align the baselines of subjective assessments (for example, pain) to be the same. When, for the i-th standardization parameter among the plurality of standardization parameters, an average is indicated by µi, a standard deviation is indicated by oi, an unstandardized feature is indicated by x, and a standardized feature for the standardization parameter i is indicated by x'i, x'i is calculated by x'i=(x-µi)/σi. The plurality of pieces of standardized feature data include a plurality of pieces of first standardized feature data obtained by standardizing the feature data by each of the plurality of first standardization parameters, a plurality of pieces of second standardized feature data obtained by standardizing the feature data by each of the plurality of second standardization parameters, ..., and a plurality of pieces of n-th standardized feature data obtained by standardizing the feature data by each of the plurality of n-th standardization parameters.

For example, if twenty first standardization parameters are extracted from the biosignal acquired from the first modeling target object, the plurality of pieces of first standardized feature data include feature data standardized by a first parameter among the first standardized parameters, feature data standardized by a second parameter among the first standardized parameters, ..., and feature data standardized by a twentieth parameter among the first standardized parameters.

In this way, by standardizing the feature data by the plurality of standardized parameters, the amount of data inputted to each of the plurality of models can be increased.

Such a plurality of pieces of standardized feature data are inputted to each of the plurality of models, and a plurality of obtained outputs are used to estimate the subjective assessment by the estimation target object by way of the procedure described above.

For example, the estimation means 121 can estimate the subjective assessment by the estimation target object using the plurality of outputs from the pieces of feature data standardized by the plurality of standardization parameters by the following procedure.

First, by inputting pieces of standardized feature data to each of a plurality of models, the estimation means 121 obtains a plurality of outputs of each of the plurality of models. The plurality of outputs of the plurality of models can include a plurality of first outputs obtained by inputting the plurality of pieces of first standardized feature data to the first model, a plurality of second outputs obtained by inputting the plurality of pieces of second standardized feature data to the second model, ..., and a plurality of n-th outputs obtained by inputting the plurality of pieces of n-th standardized feature data to the n-th model. Secondly, the estimation means 121 obtains a plurality of ensemble average outputs by taking an ensemble average of the outputs for each of the plurality of models. For example, the estimation means 121 can obtain a first ensemble average output by taking an ensemble average of the plurality of first outputs, can obtain a second ensemble average output by taking an ensemble average of the plurality of second outputs, ..., and can obtain an n-th ensemble average output by taking an ensemble average of the plurality of n-th outputs. Determining the ensemble average may be merely determining an average value of the plurality of outputs, or may be determining a weighted average of the plurality of outputs using a BIC (Bayesian information criterion). An arbitrary information criterion can be used instead of the BIC. Thirdly, the estimation means 121 takes an ensemble average of the plurality of ensemble average outputs. For example, the estimation means can take an ensemble average of the first ensemble average output, the second ensemble average output, ..., and the n-th ensemble average output. Determining the ensemble average may be merely determining an average value of the ensemble average outputs, or may be determining a weighted average of the ensemble average outputs using a BIC (Bayesian information criterion). An arbitrary information criterion can be used instead of the BIC. Fourthly, the estimation means 121 determines a score (for example, a pain score) indicating the subjective assessment based on the ensemble average.

In one embodiment, the estimation means 121 can select at least one of the plurality of feature templates or at least one of the plurality of models that are used to estimate the subjective assessment made by the estimation target object, based on no-load feature data received by the reception means 110, and estimate the subjective assessment by the estimation target object based on the feature data, and the at least one of the plurality of the feature templates or the at least one of the plurality of models that has been selected. The selected model or feature template can be selected as a model that does not easily cause a false positive of judging that a pain has occurred though it has not occurred, to occur.

The selected model or feature template can be selected as a model that does not easily cause a false positive of judging that a pain has occurred though it has not occurred, to occur. Thereby, the selected model or feature template can be a model that can clearly estimate being painful when there is a pain. For example, the selection can be performed, with such that has a high correlation coefficient between test data and a correct label (for example, 0.5 or more) as a reference. The selection may be performed by visual inspection by human eyes in the end.

Even if a model that does not easily cause a false positive is selected, it may be estimated, when there is noise, that there is pain though there is not. In order to avoid such a situation, the model can be adjusted so that the pain score does not increase at least for data during rest. For example, it is possible to select, from among eighty pain scores, a combination of a model and a standardization parameter of 0, which indicates that there is no reaction during rest. By doing so, it becomes possible to suppress occurrence of a false positive and expect improvement of the accuracy.

Though it has been described in the above example that n feature templates extracted from n modeling target objects or corresponding n models are used, the number of feature templates or corresponding models is not limited to n (n is an integer equal to or larger than 2). The number of feature templates can be an arbitrary number between 2 and n, including 2 and n, as long as the feature templates include the first feature template extracted from the first biosignal acquired from the first modeling target object and the second feature template extracted from the second biosignal acquired from the second modeling target object. The number of the plurality of models can be an arbitrary number between 2 and n, including 2 and n, as long as the plurality of models include the first model that has learned the first feature template extracted from the first biosignal acquired from the first modeling target object and the second model that has learned the second feature template extracted from the second biosignal acquired from the second modeling target object.

For example, if less than n feature templates or corresponding models exist for the n modeling target objects, one of at least one or more feature templates or corresponding models can be created from biosignals acquired from a plurality of model objects. For example, if (n-1) feature templates exist for the n modeling target objects, the (n-1) feature templates include the first feature template extracted from the first biosignal acquired from the first modeling target object, the second feature template extracted from the second biosignal acquired from the second modeling target object, ..., the (n-2)th feature template extracted from the (n-2)th biosignal acquired from the (n-2)th modeling target object, the (n-1)th biosignal acquired from the (n-1)th modeling target object and the (n-1)th feature template extracted from the n-th biosignal extracted from the n-th biosignal. For example, if (n-1) models exist for the n modeling target objects, the (n-1) models include the first model that has learned the first feature template extracted from the first biosignal acquired from the first modeling target object, the second model that has learned the second feature template extracted from the second biosignal acquired from the second modeling target object, ..., the (n-2)th model that has learned the (n-2)th feature template extracted from the (n-2)th biosignal acquired from the (n-2)th modeling target object, the (n-1)th feature template extracted from the (n-1)th biosignal acquired from the (n-1)th modeling target object and the (n-1)th model that has learned the n-th feature template extracted from the n-th biosignal extracted from the n-th biosignal.

Though each component of the system 100 is provided in the system 100 in the example described above, the present disclosure is not limited thereto. Any of the components of the system 100 can be provided outside the system 100 or remotely. For example, when the processor 120 and the storage means 130 are configured with separate hardware parts, the hardware parts may be connected via an arbitrary network. When the database of the storage means 130 is connected to another component of the system 100 via a network, the database can be configured as a storage on a cloud. In this case, the type of the network does not matter. For example, the hardware parts may be connected via a LAN and may be wirelessly or wiredly connected. The system 100 is not limited to a particular hardware configuration. For example, it is within the scope of the present disclosure to configure the processor 120 not with a digital circuit but with an analog circuit. The configuration of the system 100 is not limited to the configurations described above as long as the functions of the system 100 can be realized.

FIG. 5 is a diagram showing an example of a process 500 by the system 100 for estimating a subjective assessment made by an estimation target object.

The process 500 is executed by the processor 120 of the system 100. As described above, the storage means 130 of the system 100 stores the plurality of feature templates extracted from the plurality of biosignals acquired from the plurality of modeling target objects including the first modeling target object and the second modeling target object or the plurality of models that have learned the feature templates. The plurality of feature templates include the first feature template extracted from the first biosignal acquired from the first modeling target object and the second feature template extracted from the second biosignal acquired from the second modeling target object. The plurality of models include the first model that has learned the first feature template and the second model that has learned the second feature template.

At step S501, the processor 120 receives feature data of a biosignal acquired from an estimation target object. The processor 120 receives, for example, the feature data that has been received by the reception means 110 of the system 100, from the reception means 110. The processor 120 receives, for example, the feature data extracted from the biosignal that has been received by the reception means 110 of the system 100. At this time, the processor 120 itself may extract the feature data from the biosignal, or another means may extract the feature data from the biosignal.

At step S502, the estimation means 121 of the processor 120 estimates a subjective assessment made by the estimation target object, based on the feature data received at step S501, and the plurality of feature templates or the plurality of models.

In one embodiment, at step S502, the estimation means 121 of the processor 120 estimates the subjective assessment made by the estimation target object, based on the feature data received at step S501 and the plurality of feature templates.

For example, the estimation means 121 can acquire a plurality of correlation coefficient sets by correlating the feature data received at step S501 with each of the plurality of feature templates and estimate the subjective assessment by the estimation target object based on the plurality of correlation coefficient sets.

For example, the estimation means 121 can estimate the subjective assessment by the estimation target object using the plurality of correlation coefficient sets by the following procedure.

First, the estimation means 121 identifies, for each of the plurality of correlation coefficient sets, a value indicating a subjective assessment that is associated with a sample corresponding to the highest correlation coefficient. For example, a value indicating a subjective assessment associated with a sample corresponding to the highest correlation coefficient in a first correlation coefficient set, a value indicating a subjective assessment associated with a sample corresponding to the highest correlation coefficient in a second correlation coefficient set, ..., and a value indicating a subjective assessment associated with a sample corresponding to the highest correlation coefficient in an n-th correlation coefficient set are identified. Secondly, the estimation means 121 takes an ensemble average of the values indicating the subjective assessments of the plurality of correlation coefficient sets. For example, the estimation means 121 can take an ensemble average of the value indicating the subjective assessment of the first correlation coefficient set, the value indicating the subjective assessment of the second correlation coefficient set, ..., and the value indicating the subjective assessment of the n-th correlation coefficient set. Thirdly, the estimation means 121 determines a score indicating the subjective assessment based on the ensemble average.

In another example, the estimation means 121 can estimate the subjective assessment by the estimation target object using the plurality of correlation coefficient sets by the following procedure.

First, the estimation means 121 identifies, for each of the plurality of correlation coefficient sets, values indicating subjective assessments that are associated with samples corresponding to a top plurality of correlation coefficients. For example, values indicating subjective assessments associated with samples corresponding to a top plurality of correlation coefficients in the first correlation coefficient set, values indicating subjective assessments associated with samples corresponding to a top plurality of correlation coefficients in the second correlation coefficient set, ..., and values indicating subjective assessments associated with samples corresponding to a top plurality of correlation coefficients in the n-th correlation coefficient set are identified. Secondly, for each of the correlation coefficient sets, the estimation means 121 obtains an ensemble average correlation coefficient by taking an ensemble average of the values indicating the top plurality of subjective assessment. For example, the estimation means 121 can obtain a first ensemble average correlation coefficient by taking an ensemble average of the values of the subjective assessments of the first correlation coefficient sets, can obtain a second ensemble average correlation coefficient by taking an ensemble average of the values of the subjective assessments of the second correlation coefficient sets, ..., and can obtain an n-th ensemble average correlation coefficient by taking an ensemble average of the values of the subjective assessments of the n-th correlation coefficient sets. Thirdly, the estimation means 121 takes an ensemble average of the ensemble average correlation coefficients of the plurality of correlation coefficient sets. For example, the estimation means 121 can take an ensemble average of the first ensemble average correlation coefficient, the second ensemble average correlation coefficient, ..., and the n-th ensemble average correlation coefficient. Fourthly, the estimation means 121 determines a score indicating the subjective assessment based on the ensemble average.

In another embodiment, at step S502, the estimation means 121 of the processor 120 estimates the subjective assessment made by the estimation target object, based on the feature data received at step S501 and the plurality of models.

For example, the estimation means 121 can acquire a plurality of outputs by inputting the feature data received at step S501 to each of the plurality of models, and estimate the subjective assessment by the estimation target object based on the plurality of outputs.

For example, the estimation means 121 can estimate the subjective assessment by the estimation target object using the plurality of outputs by the following procedure.

First, the estimation means 121 takes an ensemble average of the plurality of outputs. Determining the ensemble average may be merely determining an average value of the plurality of outputs, or may be determining a weighted average of the plurality of outputs using a BIC (Bayesian information criterion). An arbitrary information criterion can be used instead of the BIC. Secondly, the estimation means 121 determines a score indicating the subjective assessment based on the ensemble average.

By the process 500, the system 100 can output data showing time-series change in the subjective assessment by the estimation target object. The data showing the time-series change is outputted to the outside of the system 100 via the output means 140. A user of the system 100 can understand what is the subjective assessment by the estimation target object by interpreting the data.

At this time, the process 500 need not give a load to the estimation target object in advance. Therefore, according to the process 500 of the system of the present disclosure, it is possible to highly accurately predict the subjective assessment by the estimation target object without giving a load to the estimation target object in advance. Since giving a load to the estimation target object is a burden on the estimation target object, it is useful that it is not necessary to give a load in advance.

The system 100 for estimating a subjective assessment made by an estimation target object can be used to estimate a pain experienced by the estimation target object in one example.

FIG. 6 shows an example of a process 600 for estimating a pain experienced by an estimation target object by the system 100 for estimating a subjective assessment made by an estimation target object;

The process 600 is executed by the processor 120 of the system 100. As described above, the storage means 130 of the system 100 stores the plurality of feature templates extracted from the plurality of biosignals acquired from the plurality of modeling target objects including the first modeling target object and the second modeling target object or the plurality of models that have learned the feature templates. The plurality of biosignals are a plurality of biosignals at a time when a stimulus is given to the plurality of modeling target objects; the first biosignal is a first biosignal at the time when the stimulus is given to the first modeling target object; and the second biosignal is a second biosignal at the time when the stimulus is given to the second modeling target object. The plurality of feature templates include the first feature template extracted from the first biosignal acquired from the first modeling target object and the second feature template extracted from the second biosignal acquired from the second modeling target object. The plurality of models include the first model that has learned the first feature template and the second model that has learned the second feature template.

At step S601, the processor 120 receives feature data of a biosignal at a time when a stimulus is given to an estimation target object. The processor 120 receives, for example, the feature data that has been received by the reception means 110 of the system 100, from the reception means 110. The processor 120 receives, for example, the feature data extracted from the biosignal that has been received by the reception means 110 of the system 100. At this time, the processor 120 itself may extract the feature data from the biosignal, or another means may extract the feature data from the biosignal.

At step S602, the estimation means 121 of the processor 120 estimates a pain experienced by the estimation target object, based on the feature data received at step S601, and the plurality of feature templates or the plurality of models. The pain experienced by the estimation target object is a pain that the estimation target object feels by the stimulus given to the estimation target object.

In one embodiment, at step S602, the estimation means 121 of the processor 120 estimates the pain experienced by the estimation target object, based on the feature data received at step S601 and the plurality of feature templates.

The estimation means 121 can generate a correlation matrix by correlating the feature data received at step S601 with each of the plurality of feature templates and estimate the pain experienced by the estimation target object based on the correlation matrix.

For example, the estimation means 121 can estimate the subjective assessment by the estimation target object using a plurality of correlation coefficient sets by the following procedure.

First, the estimation means 121 identifies, for each of the plurality of correlation coefficient sets, a value indicating a subjective assessment that is associated with a sample corresponding to the highest correlation coefficient. The value indicating a subjective assessment can be, for example, the value of a COVAS label associated with each sample. For example, the value of a COVAS label associated with a sample corresponding to the highest correlation coefficient in a first correlation coefficient set (a first COVA label value), the value of a COVAS label associated with a sample corresponding to the highest correlation coefficient in a second correlation coefficient set (a second COVA label value), ..., and the value of a COVAS label associated with a sample corresponding to the highest correlation coefficient in an n-th correlation coefficient set (an n-th COVA label value) are identified. Secondly, the estimation means 121 takes an ensemble average of the values indicating the subjective assessments of the plurality of correlation coefficient sets. For example, the estimation means 121 can take an ensemble average of the first COVA label value, the second COVA label value, ..., and the n-th COVA label value. Determining the ensemble average may be merely determining an average value of the COVA label values, or may be determining a weighted average of the COVA label values using a BIC (Bayesian information criterion). An arbitrary information criterion can be used instead of the BIC. Thirdly, the estimation means 121 determines a score (for example, a pain score) indicating the subjective assessment based on the ensemble average.

In another example, the estimation means 121 can estimate the subjective assessment by the estimation target object using the plurality of correlation coefficient sets by the following procedure.

First, the estimation means 121 identifies, for each of the plurality of correlation coefficient sets, values indicating subjective assessments that are associated with samples corresponding to a top plurality of correlation coefficients. The values indicating subjective assessments can be, for example, the values of COVAS labels associated with the samples. For example, the values of COVAS labels associated with samples corresponding to a top plurality of correlation coefficients in the first correlation coefficient set (a first COVA label value group), the values of COVAS labels associated with samples corresponding to a top plurality of correlation coefficients in the second correlation coefficient set (a second COVA label value group), ..., and the values of COVAS labels associated with samples corresponding to a top plurality of correlation coefficients in the n-th correlation coefficient set (an n-th COVA label value group) are identified. As for the samples of the top plurality of values indicating subjective assessments, such indexes of values of COVAS labels that the error is below 0.5 are extracted using AdaBoost, and the indexes may be selected. For example, the samples of the top plurality of values indicating subjective assessments can be selected so that, when a pain score during rest or at the time of imposing a task of intentionally causing noise to occur (a noise session) on an estimation target object is assumed to be 0 indicating that pain has not occurred at all, occurrence of a false positive, which is an error of judging that a pain has occurred though it has not occurred, is reduced as long as possible. Secondly, for each of the correlation coefficient sets, the estimation means 121 obtains an ensemble average correlation coefficient by taking an ensemble average of the values indicating the top plurality of subjective assessment. For example, the estimation means 121 can obtain a first ensemble average correlation coefficient by taking an ensemble average for the first COVA label value group, can obtain a second ensemble average correlation coefficient by taking an ensemble average for the second COVA label value group, ..., and can obtain an n-th ensemble average correlation coefficient by taking an ensemble average for the n-th COVA label value group. Determining the ensemble average may be merely determining an average value of the COVA label values, or may be determining a weighted average of the COVA label values using a BIC (Bayesian information criterion). An arbitrary information criterion can be used instead of the BIC. Thirdly, the estimation means 121 takes an ensemble average of the ensemble average correlation coefficients of the plurality of correlation coefficient sets. For example, the estimation means 121 can take an ensemble average of the first ensemble average correlation coefficient, the second ensemble average correlation coefficient, ..., and the n-th ensemble average correlation coefficient. Determining the ensemble average may be merely determining an average value of the ensemble average correlation coefficients, or may be determining a weighted average of the ensemble average correlation coefficients using a BIC (Bayesian information criterion). An arbitrary information criterion can be used instead of the BIC. Fourthly, the estimation means 121 determines a score (for example, a pain score) indicating the subjective assessment based on the ensemble average.

In another embodiment, at step S602, the estimation means 121 of the processor 120 estimates the pain experienced by the estimation target object, based on the feature data received at step S601 and the plurality of models.

For example, the estimation means 121 can acquire a plurality of outputs by inputting the feature data received at step S601 to each of the plurality of models, and estimate the pain experienced by the estimation target object based on the plurality of outputs.

For example, the estimation means 121 can estimate the subjective assessment by the estimation target object using the plurality of outputs by the following procedure.

First, the estimation means 121 takes an ensemble average of the plurality of outputs. Determining the ensemble average may be merely determining an average value of the plurality of outputs, or may be determining a weighted average of the plurality of outputs using a BIC (Bayesian information criterion). An arbitrary information criterion can be used instead of the BIC. Secondly, the estimation means 121 determines a score (for example, a pain score) indicating the subjective assessment based on the ensemble average.

By the process 600, the system 100 can output data showing time-series change in the pain experienced by the estimation target object. The data showing the time-series change is outputted to the outside of the system 100 via the output means 140. The user of the system 100 can understand what is the pain that the estimation target object has felt by interpreting the data.

At this time, the process 600 need not give a stimulus to the estimation target object in advance. Therefore, according to the process 600 of the system of the present disclosure, it is possible to highly accurately predict the pain experienced by the estimation target object without giving a stimulus to the estimation target object in advance. Since giving a stimulus to the estimation target object is a burden on the estimation target object, it is useful that it is unnecessary to give a stimulus in advance.

Though it has been described in the above examples that the steps of each of the processes 500 and 600 are performed in particular order, the described order is a mere example. The steps of each of the processes 500 and 600 can be performed in arbitrary order that is logically possible. Further, instead of each of the described steps or in addition to each of the described steps, other steps can be included.

Though, in the examples described above with reference to FIGS. 5 and 6, the process of each step shown in FIGS. 5 and 6 is described as being realized by the processor 120 and the program stored in the memory, the present disclosure is not limited thereto. At least one of the processes of the steps of each of FIGS. 5 and 6 may be realized by a hardware component such as a control circuit.

The present disclosure has been described above by showing embodiments preferred to facilitate understanding. The present disclosure will be described below based on examples. However, the above description and the examples below are provided only for the purpose of exemplification and are not provided for the purpose of limiting the present disclosure. Therefore, the scope of the present disclosure is not limited by the embodiments or the examples, which are specifically described in the present specification, but is limited only by the claims.

### EXAMPLES

The examples will be described below. Handling of an estimation target object used in the examples below is in compliance with standards specified by Osaka University when necessary, and in compliance with Declaration of Helsinki and ICH-GCP when clinical researches are involved.

### (Example 1)

In the present example, experiments for estimating a pain that an estimation target object felt when a thermal stimulus was given to the estimation target object were conducted using feature templates generated using pieces of brain wave data acquired when a thermal stimulus was given to a plurality of modeling target objects. A method and the like are shown below.

### (Protocol for estimation target object)

The estimation target object was a healthy 27-year-old female.

First, brain wave electrodes are attached to the estimation target object. Disinfected electrodes were attached to the thumb of the left foot and the thumb of the left hand. Then, disinfected electrodes were attached to the middle and ring fingers of the left hand. Next, a "noise session" was started for the estimation target object. The noise session is a session for measuring brain wave data to which noise is artificially added, by causing an estimation target object to perform each of the following four tasks three times in about three minutes.
(1) Close the eyes strongly.
(2) Rotate the cheeks by hands.
(3) Shake the head left and right.
(4) Turn over left and right (in the case of conducting experiments on a bed).
By discriminating a brain wave with noise by machine learning, it becomes possible to omit a state when there is noise.

The estimation target object was instructed to close her eyes and not to move her body as far as possible.

Next, the first thermal stimulus was given to the arm of the estimation target object. The first thermal stimulus was given such that the temperature shown in FIG. 7 is achieved, in order to acquire learning data for creation of a model.

After the first thermal stimulus ends, the second thermal stimulus was given to the arm of the estimation target object. The second thermal stimulus was also given such that the temperature shown in FIG. 7 is achieved, in order to acquire learning data for creation of a model.

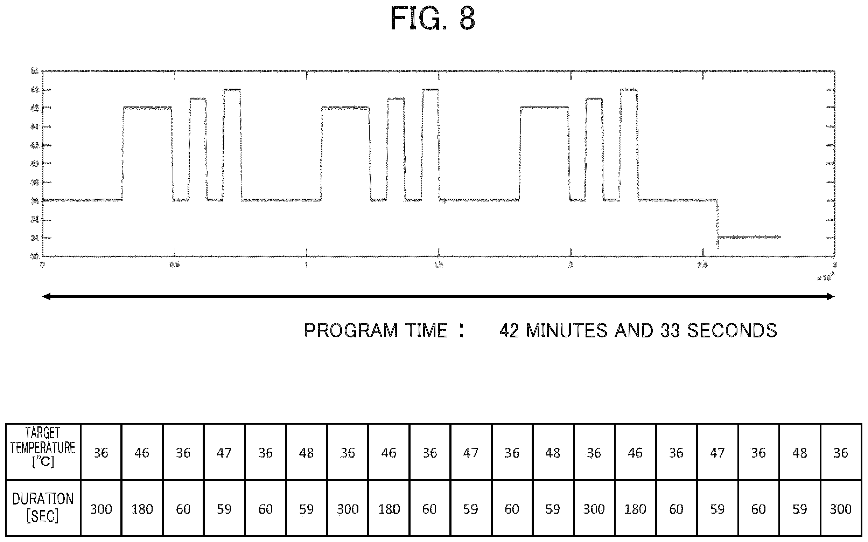

After that, the third thermal stimulus was given to the arm of the estimation target object. The third thermal stimulus was given such that the temperature shown in FIG. 8 is achieved, in order to acquire test data.

### (Used feature templates)

Each feature template was created by the following procedure.
1. By giving each modeling target object such a heat stimulus that temperature rises from 36°C to 49°C and drops in steps twice (see FIG. 7) in advance, obtained brain wave data of pain and labels (a trigger and a COVAS template) were read. The COVAS template was created by, after experiments of the n modeling target objects and before creation of the feature templates, performing addition averaging of pieces of data corresponding to the n modeling target objects and fitting the scale to 0 to 100.
2. First, as preprocessing, a total of 18-ch time-series brain wave data, that is, 6-ch brain wave data (monopolar) of the forehead, 6-ch bipolar data derived using the 6-ch brain wave data and a 6-ch CAR (common average reference) was extracted.
3. After applying noise processing using a trapezoidal filter, N samples were cutout by shifting a 16-second window (a large window) by one second each time for the total length of each piece of time-series brain wave data. The size is 16000 samples×N when the sampling frequency is assumed to be 1000 Hz. A similar process was also performed for the COVAS template labels. Each new COVAS label was defined by an average value of the cut-out window size (16000 samples) so that the new COVAS labels correspond to N brain wave samples, respectively, one-to-one. At this time, the size of the COVAS labels is 1×N.
4. Inside each cut-out large window, nine different sequences to be time information were defined by further shifting an 8-second window (a small window) by one second each time. It is because LSTM is strong in processing in consideration of time information that the sequences are defined.
5. For one small window, a process for extracting the following four kinds of features (amplitude, frequency power, coherence and entropy) was performed. For the frequency power and the coherence, eight frequency bands are defined, that is, δ(2-5 Hz), θ(5-8 Hz), α(8-14 Hz), β(14-28 Hz), γ(28-58 Hz), γ2(62-118 Hz), γ3(122-178 Hz) and γ4(182-238 Hz).
   (1) Amplitude: 18 amplitudes were extracted for 18 ch.
   (2) Frequency power: 144 frequency powers of 8 frequency bands were extracted for 18 ch.
   (3) Coherence: 144 coherences of 8 frequency bands were extracted for 18 ch.
   (4) Entropy: 18 entropies were extracted for 18 ch. A total of 324 features were extracted.
6. At this point of time, three-dimensional data of brain wave features N×9×324, and 1×N COVAS labels have been extracted. The three-dimensional data has a data structure used at the time of creating the LSTM model described above. At the time of performing feature template matching for test data, the nine pieces of sequence information are not necessary. Therefore, only the first sequence is used. The data structure at that time is two-dimensional data of N×324.
7. As experiment data, data during rest (rest) and data at a time when a heat stimulus is given (heat) exist. It is known that data obtained by combining both data in an appropriate proportion is effective as learning data. For example, by using a COVAS label as a heat label and using such a label that all the values are 0 as a rest label, it becomes possible to express strength/weakness of pain including the pain during rest.
8. A feature template for one person is defined by combining rest and heat data. What is obtained by combining pieces of data from which features have been extracted, for n persons is an overall large feature template, in which labels corresponding to the pieces of data from which features have been extracted, respectively, one-to-one are also completely included.

### (Results)

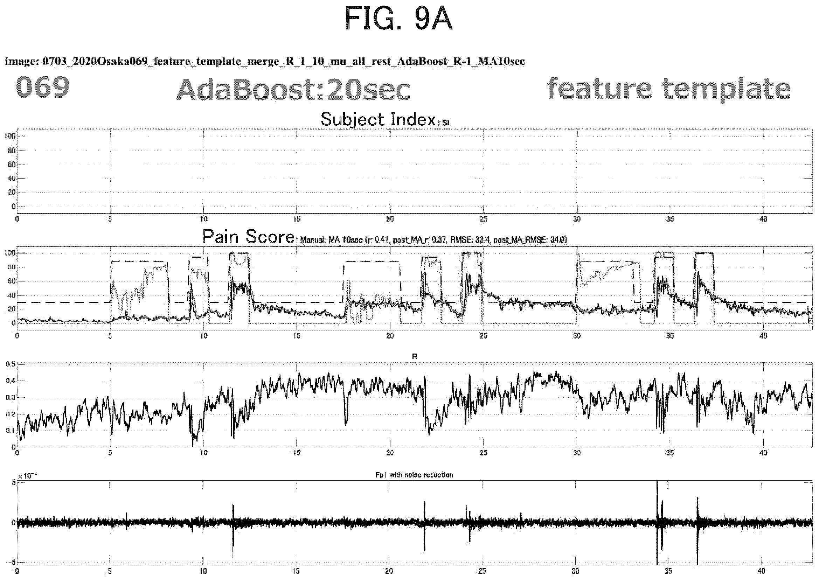

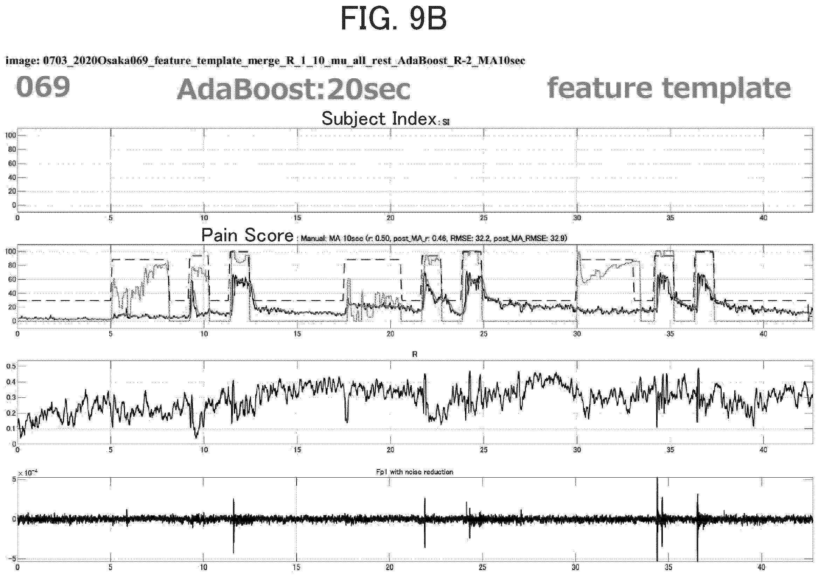

FIGS. 9A and 9B show results in the example 1.

In each of FIGS. 9A and 9B, the first graph from the bottom shows brain wave data acquired from an estimation target object F069. In each of FIGS. 9A and 9B, the second graph from the bottom shows an output at the time of correlating a feature of the brain data acquired from the estimation target object F069 with the feature template. In the third graph from the bottom, (1) the black solid line indicates pain scores; (2) the gray solid line indicates 10-sec moving averages of the pain scores; (3) the dotted line indicates subjective assessments (CoVAS) of the subject; and (4) the broken line indicates temperature sections (three sections of each of 46°C, 47°C and 48°C). A difference between FIGS. 9A and 9B is whether the feature template for estimating a pain score was created with the first thermal stimulus or with the second thermal stimulus. Both of results of the pain scores of both diagrams indicate ensemble averages of pain scores estimated from the plurality of feature templates.

As seen from the result of FIG. 9, it can be observed that, in the temperature sections corresponding to 46°C, the pain score does not increase though the subjective assessment (CoVAS) of the subject increases; but, in the temperature sections corresponding to 47°C and 48°C, CoVAS and the pain score increase together. This shows that pains in response to thermal stimuli of high temperatures 47°C and 48°C can be appropriately expressed. Further, for both of the feature template created with the first thermal stimulus and the feature template created with the second thermal stimulus, the results show almost the same tendency. It can be thought that a feature template is reproducible under the same conditions.

### (Example 2)

In the present example, experiments for estimating a pain that an estimation target object felt when a thermal stimulus was given to the estimation target object were conducted using LSTM models generated using pieces of brain wave data acquired when thermal stimuli was given to a plurality of modeling target objects.

### (Protocol for estimation target object)

The estimation target object was a healthy 27-year-old female.

First, brain wave electrodes are attached to the estimation target object. Disinfected electrodes were attached to the thumb of the left foot and the thumb of the left hand. Then, disinfected electrodes were attached to the middle and ring fingers of the left hand. Next, a "noise session" was started for the estimation target object. The noise session is a session for measuring brain wave data to which noise is artificially added, by causing an estimation target object to perform each of the following four tasks three times in about three minutes.
(1) Close the eyes strongly.
(2) Rotate the cheeks by hands.
(3) Shake the head left and right.
(4) Turn over left and right (in the case of conducting experiments on a bed).
By discriminating a brain wave with noise by machine learning, it becomes possible to omit a state when there is noise.

The estimation target object was instructed to close her eyes and not to move her body as far as possible.

Next, the first thermal stimulus was given to the arm of the estimation target object. The first thermal stimulus was given such that the temperature shown in FIG. 7 is achieved, in order to acquire learning data for creation of a model.

After the first thermal stimulus ends, the second thermal stimulus was given to the arm of the estimation target object. The second thermal stimulus was also given such that the temperature shown in FIG. 7 is achieved, in order to acquire learning data for creation of a model.

After that, the third thermal stimulus was given to the arm of the estimation target object. The third thermal stimulus was given such that the temperature shown in FIG. 8 is achieved, in order to acquire test data.

### (Used LSTM models)

1. By giving each modeling target object such a heat stimulus that temperature rises from 36°C to 49°C and drops in steps twice (see FIG. 7) in advance, obtained brain wave data of pain and labels (a trigger and a COVAS template) were read. The COVAS template was created by, after experiments of the n modeling target objects and before creation of the feature templates, performing addition averaging of pieces of data corresponding to the n modeling target objects and fitting the scale to 0 to 100.
2. First, as preprocessing, a total of 18-ch time-series brain wave data, that is, 6-ch brain wave data (monopolar) of the forehead, 6-ch bipolar data derived using the 6-ch brain wave data and a 6-ch CAR (common average reference) was extracted.
3. After applying noise processing using a trapezoidal filter, N samples were cutout by shifting a 16-second window (a large window) by one second each time for the total length of each piece of time-series brain wave data. The size is 16000 samples×N when the sampling frequency is assumed to be 1000 Hz. A similar process was also performed for the COVAS template labels. Each new COVAS label was defined by an average value of the cut-out window size (16000 samples) so that the new COVAS labels correspond to N brain wave samples, respectively, one-to-one. At this time, the size of the COVAS labels is 1×N.
4. Inside each cut-out large window, nine different sequences to be time information were defined by further shifting an 8-second window (a small window) by one second each time. It is because LSTM is strong in processing in consideration of time information that the sequences are defined.
5. For one small window, a process for extracting the following four kinds of features (amplitude, frequency power, coherence and entropy) was performed. For the frequency power and the coherence, eight frequency bands are defined, that is, δ(2-5 Hz), θ(5-8 Hz), α(8-14 Hz), β(14-28 Hz), γ(28-58 Hz), γ2(62-118 Hz), γ3(122-178 Hz) and γ4(182-238 Hz).
   (1) Amplitude: 18 amplitudes were extracted for 18 ch.
   (2) Frequency power: 144 frequency powers of 8 frequency bands were extracted for 18 ch.
   (3) Coherence: 144 coherences of 8 frequency bands were extracted for 18 ch.
   (4) Entropy: 18 entropies were extracted for 18 ch. A total of 324 features were extracted.
6. At this point of time, three-dimensional data of brain wave features N×9×324, and 1×N COVAS labels have been extracted. The three-dimensional data has a data structure used at the time of creating the LSTM model described above. At the time of performing feature template matching for test data, the nine pieces of sequence information are not necessary. Therefore, only the first sequence is used. The data structure at that time is two-dimensional data of N×324.
7. As experiment data, data during rest (rest) and data at a time when a heat stimulus is given (heat) exist. It is known that data obtained by combining both data in an appropriate proportion is effective as learning data. For example, by using a COVAS label as a heat label and using such a label that all the values are 0 as a rest label, it becomes possible to express strength/weakness of pain including the pain during rest.
8. A feature template for one person is defined by combining rest and heat data. What is obtained by combining pieces of data from which features have been extracted, for n persons is an overall large feature template, in which labels corresponding to the pieces of data from which features have been extracted, respectively, one-to-one are also completely included. By causing LSTM to perform learning using the three-dimensional data of brain wave features N×9×324 and the 1×N COVAS labels, a model is created. At this time, twenty standardization parameters are stored, and, at the time of applying a model to test data, the parameters are developed to perform standardization. As for standardization parameters at the time of creating a model, all samples are used to perform standardization similarly to standardization performed in general machine learning.

### (Results)

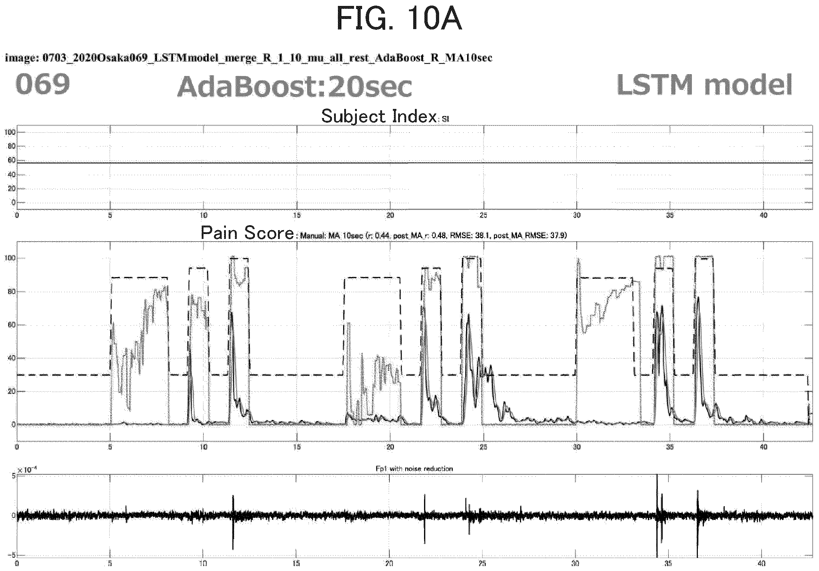

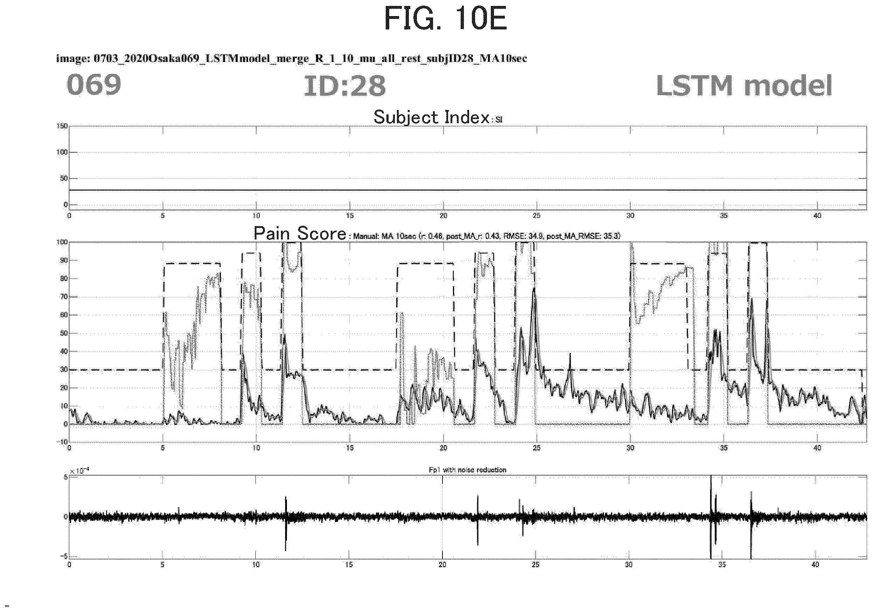

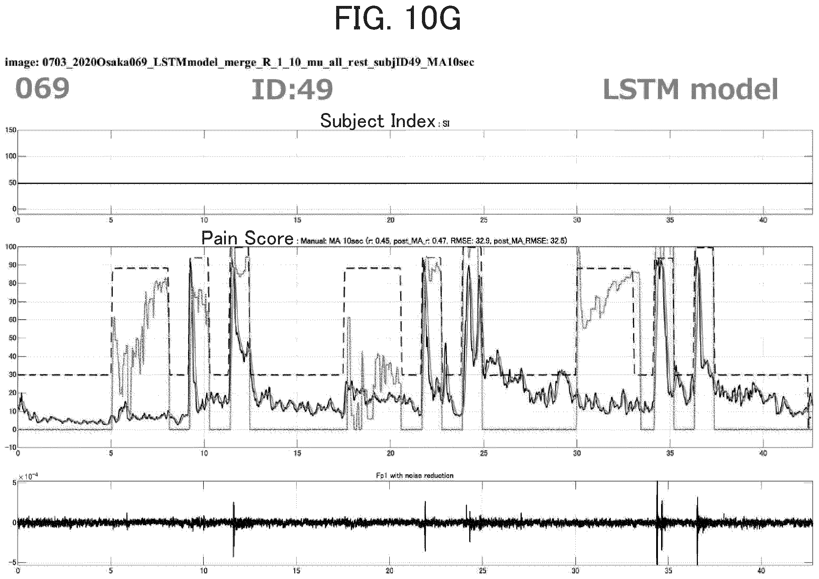

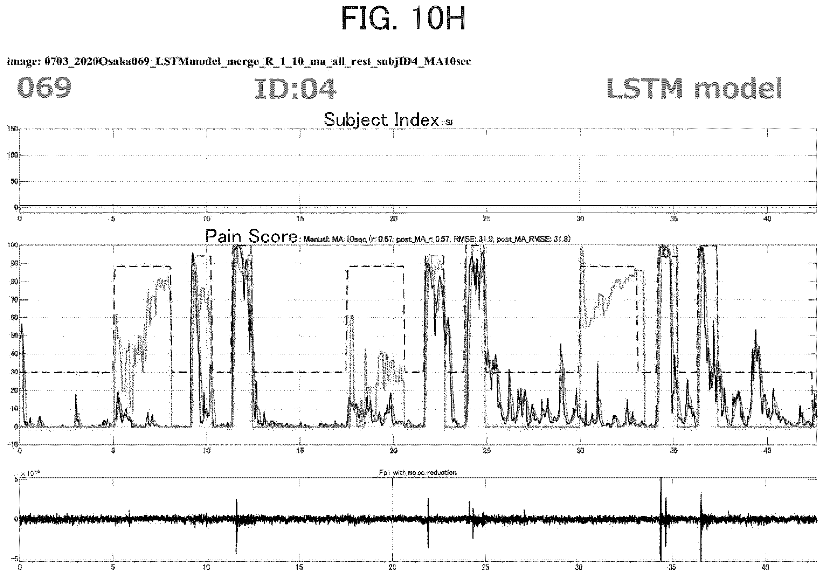

FIGS. 10A to 10I show results in the example 2.

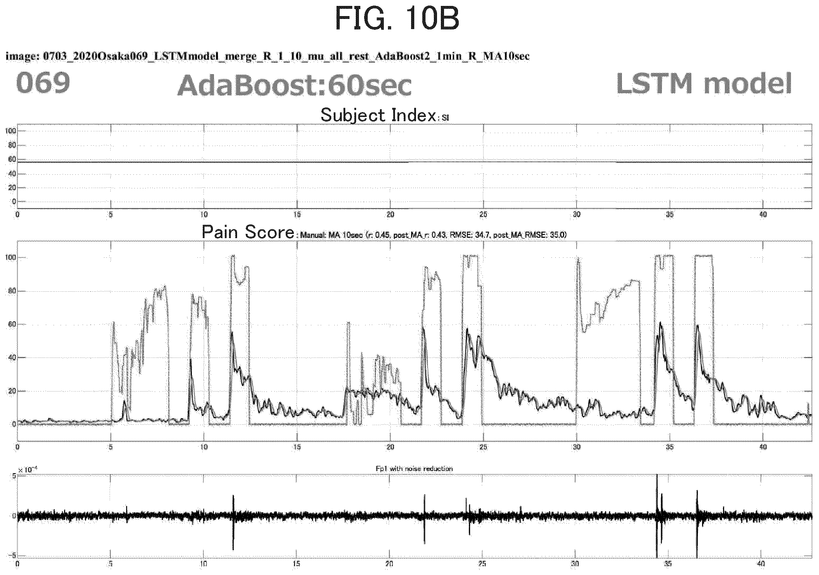

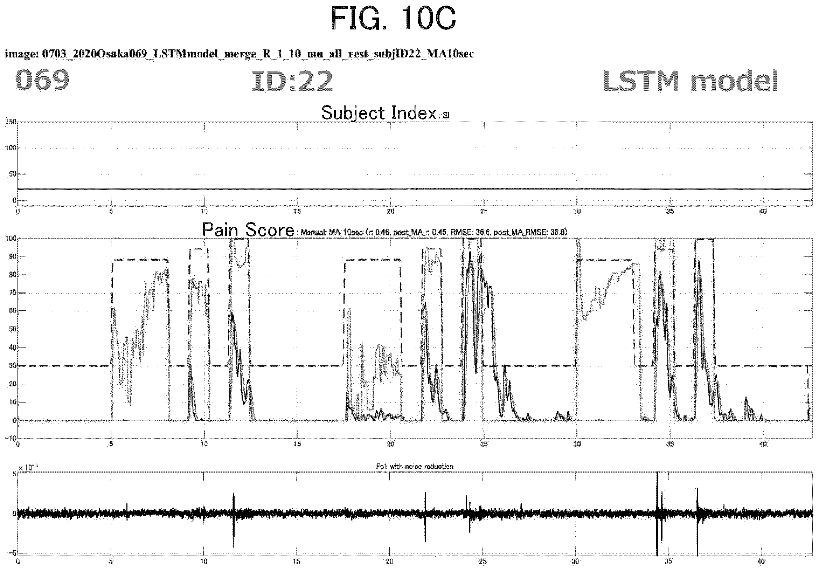

In each of FIGS. 10A to 10H, the second graph from the bottom shows an output at the time of inputting feature data of the brain data acquired from the estimation target object F069 to an LSTM model.
(1) The black solid line indicates pain scores; (2) the gray solid line indicates 10-sec moving averages of the pain scores; (3) the dotted line indicates subjective assessments (CoVAS) of the subject; and (4) the broken line indicates temperature sections (three sections of each of 46°C, 47°C and 48°C). Differences among FIGS. 10A to 10H are as follows. FIG. 10A: an ensemble average of pain scores estimated by the LSTM model (AdaBoost is applied using data during rest (without pain) for 20 seconds after start); FIG. 10B: an ensemble average of pain scores estimated by the LSTM model (AdaBoost is applied using data during rest (without pain) for 60 seconds after start); FIGS. 10C to 10H: pain scores estimated by LSTM models obtained from individual modeling target objects

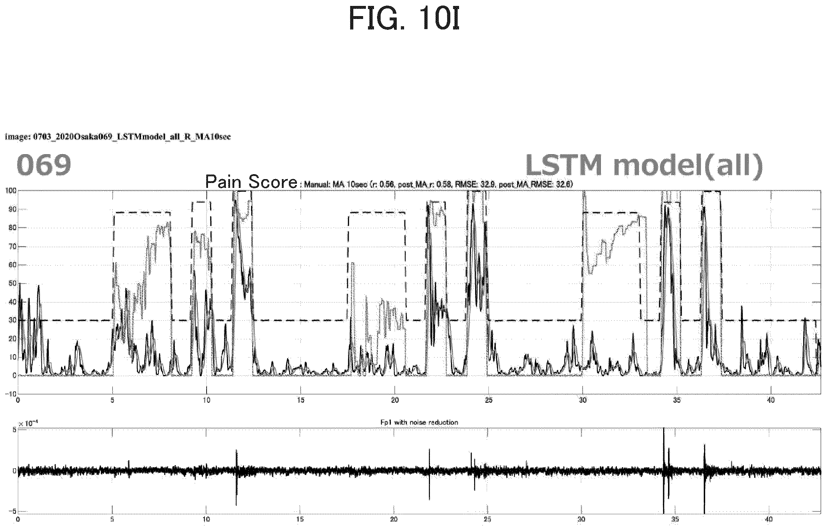

FIG. 10I shows pain scores estimated by one LSTM model created by combining pieces of feature data obtained from all of ninety modeling target objects. Outputs at a time when feature data of the brain wave data acquired from the estimation target object F069 was inputted to the created LSTM model is shown.
(1) The black solid line indicates pain scores; (2) the gray solid line indicates 10-sec moving averages of the pain scores; (3) the dotted line indicates subjective assessments (CoVAS) of the subject; and (4) the broken line indicates temperature sections (three sections of each of 46°C, 47°C and 48°C).

When FIGS. 10A and 10B are compared for the ensemble average of pain scores estimated by the LSTM model, it can be read that the rising up of pain in response to the thermal stimulus is expressed better in FIG. 10B, while persistence of the pain (lingering of the pain) for the thermal stimulus is expressed better in FIG. 10A. A difference between FIGS. 10A and 10B is the length of data during rest (without pain) to which AdaBoost is applied. It can be thought that persistence of pain (lingering of pain) can be expressed if there are many samples.

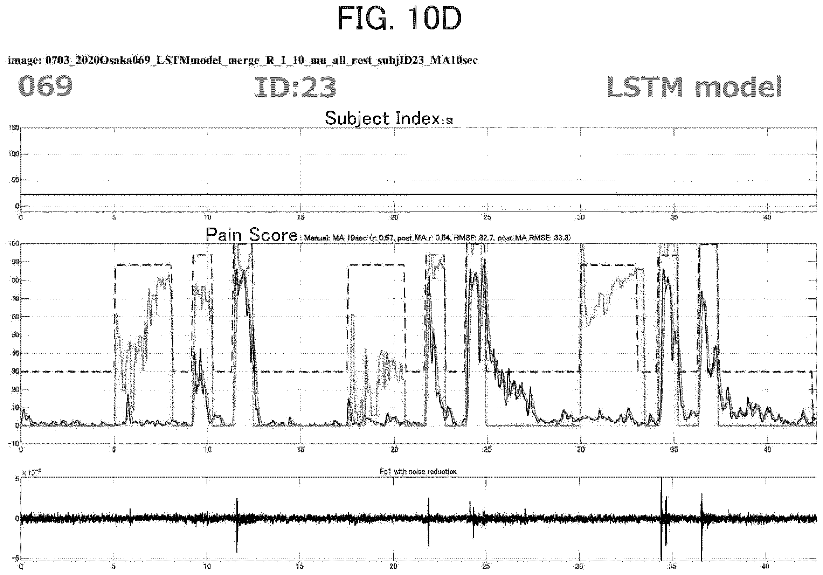

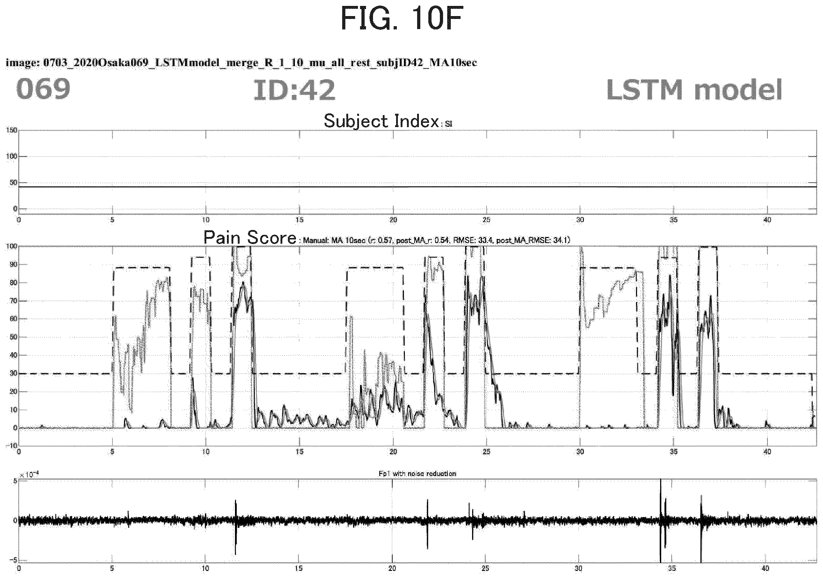

As for the pain scores estimated by the LSTM models obtained from the individual modeling target objects (FIGS. 10C to 10H), not ensemble averages but pain scores that are directly expressed in comparison with FIGS. 10A and 10B, and, therefore, it can be thought that the pain score estimation accuracy is higher than the ensemble averages if selection of the models is appropriate. For example, in comparison with FIGS. 10A and 10B, it is read that FIGS. 10D and 10F show a higher degree of matching with subjective assessment values (CoVAS) in both of the characteristics of the "rising up" and "persistence" of pain.

From FIG. 10I, it is read that, during rest at the beginning, the pain score increases though there is no pain, and the false positive rate tends to be high. From this, it can be also thought that the pain score estimation accuracy is higher by appropriately selecting an individual LSTM model by a personal filter than by an LSTM model created by combining pieces of feature data obtained from a plurality of modeling target objects.

### (Example 3)

In the present example, experiments for estimating pains that estimation target objects felt when local anesthesia was administered during operations were conducted, using an LSTM models generated using pieces of brain wave data acquired when a thermal stimulus was given to a plurality of modeling target objects.

### (Protocol for estimation target objects)

The estimation target objects were healthy 47-to-76-year-old males or females.

The estimation target objects included four patients with IDs of F023, F025, F037 and F040. The ages and genders of the patients are as shown below. F023: 47 years old; male
F025: 76 years old; female
F037: 68 years old; male
F040: 55 years old; female

Using brain wave electrodes attached to each of the estimation target objects, brain wave data before and after administering local anesthesia during an operation was acquired.

A doctor visually cut out an estimation target section from the acquired brain wave data, watching video of the operation. The time of administering local anesthesia is called a local anesthesia section (an LA section), and non-LA sections existed before and after the LA section. A rest section existed before the LA section, and a section of lingering-of-pain existed after the LA section.

### (Used LSTM models)

Ninety LSTM models were individually created by giving a thermal stimulus to ninety modeling target objects that were healthy subjects in their twentieth. After that, four champion models were selected and combined. The four LSTM models included models with IDs of ID 007, ID 043, ID 126 and ID 141.
1. By giving each modeling target object such a heat stimulus that temperature rises from 36°C to 49°C and drops in steps twice (see FIG. 7) in advance, obtained brain wave data of pain and labels (a trigger and a COVAS template) are read. The COVAS template was created by, after experiments of the n modeling target objects and before creation of the feature templates, performing addition averaging of pieces of data corresponding to the n modeling target objects and fitting the scale to 0 to 100.
2. First, as preprocessing, a total of 18-ch time-series brain wave data, that is, 6-ch brain wave data (monopolar) of the forehead, 6-ch bipolar data derived using the 6-ch brain wave data and a 6-ch CAR (common average reference) was extracted.
3. After applying noise processing using a trapezoidal filter, N samples were cutout by shifting a 16-second window (a large window) by one second each time for the total length of each piece of time-series brain wave data. The size is 16000 samples×N when the sampling frequency is assumed to be 1000 Hz. A similar process was also performed for the COVAS template labels. Each new COVAS label was defined by an average value of the cut-out window size (16000 samples) so that the new COVAS labels correspond to N brain wave samples, respectively, one-to-one. At this time, the size of the COVAS labels is 1×N.
4. Inside each cut-out large window, nine different sequences to be time information were defined by further shifting an 8-second window (a small window) by one second each time. It is because LSTM is strong in processing in consideration of time information that the sequences are defined.
5. For one small window, a process for extracting the following four kinds of features (amplitude, frequency power, coherence and entropy) was performed. For the frequency power and the coherence, eight frequency bands are defined, that is, δ(2-5 Hz), θ(5-8 Hz), α(8-14 Hz), β(14-28 Hz), γ(28-58 Hz), γ2(62-118 Hz), γ3(122-178 Hz) and γ4(182-238 Hz).
   (1) Amplitude: 18 amplitudes were extracted for 18 ch.
   (2) Frequency power: 144 frequency powers of 8 frequency bands were extracted for 18 ch.
   (3) Coherence: 144 coherences of 8 frequency bands were extracted for 18 ch.
   (4) Entropy: 18 entropies were extracted for 18 ch. A total of 324 features were extracted.
6. At this point of time, three-dimensional data of brain wave features N×9×324, and 1×N COVAS labels have been extracted. The three-dimensional data has a data structure used at the time of creating the LSTM model described above. At the time of performing feature template matching for test data, the nine pieces of sequence information are not necessary. Therefore, only the first sequence is used. The data structure at that time is two-dimensional data of N×324.
7. As experiment data, data during rest (rest) and data at a time when a heat stimulus is given (heat) exist. It is known that data obtained by combining both data in an appropriate proportion is effective as learning data. For example, by using a COVAS label as a heat label and using such a label that all the values are 0 as a rest label, it becomes possible to express strength/weakness of pain including the pain during rest.
8. A feature template for one person is defined by combining rest and heat data. What is obtained by combining pieces of data from which features have been extracted, for n persons is an overall large feature template, in which labels corresponding to the pieces of data from which features have been extracted, respectively, one-to-one are also completely included.

For each of the four LSTM models, twenty different standardization parameters were derived.

As test data, data acquired from patients including the 76-year-old patient was used.

### (Data processing)

(A) From the plurality of LSTM models, a part of the models are selected.
(B) From twenty models existing inside each of the models selected at (A), a part of the models are selected.
(C) From ensemble averages of the model groups selected at (B), a pain score is calculated

### (Results)

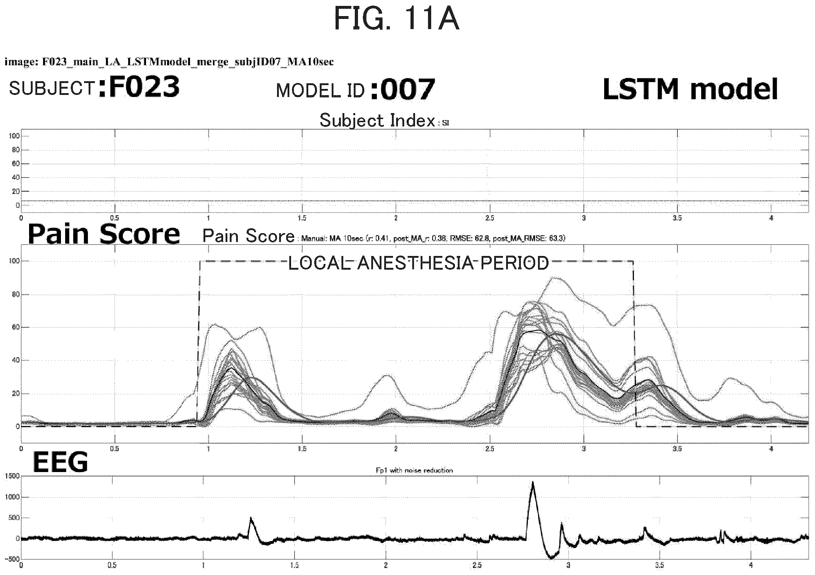

FIGS. 11A to 11P show results in the example 3.

In each of FIGS. 11A to 11D, a graph at a lower part shows brain wave data acquired from the estimation target object F023. In each of FIGS. 11A to 11D, graphs in the middle show outputs at the time of inputting the brain wave data acquired from the estimation target object F023 to the models with the ID 007, ID 043, ID 126 and ID 141, respectively.
(1) The light-colored lines indicate the twenty pain scores; (2) the black line indicates ensemble average values of (1); and (3) the dark-colored line indicates results of 10-sec moving averages of (2). The twenty pain scores correspond to pieces of data for which the twenty standardization parameters are used, respectively.

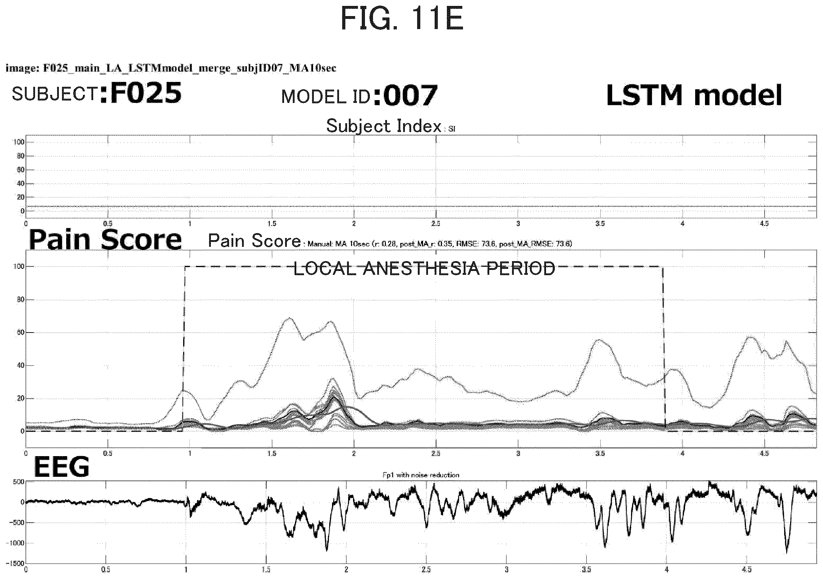

In each of FIGS. 11E to 11H, a graph at a lower part shows brain wave data acquired from the estimation target object F025. In each of FIGS. 11E to 11H, graphs in the middle show outputs at the time of inputting the brain wave data acquired from the estimation target object F025 to the models with the ID 007, ID 043, ID 126 and ID 141, respectively. (1) The light-colored lines indicate the twenty pain scores; (2) the black line indicates ensemble average values of (1); and (3) the dark-colored line indicates results of 10-sec moving averages of (2). The twenty pain scores correspond to pieces of data for which the twenty standardization parameters are used, respectively.

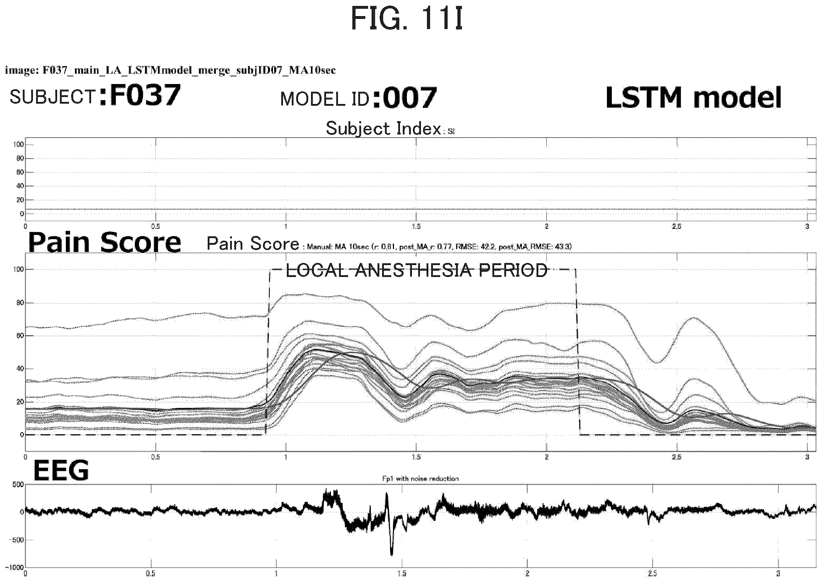

In each of FIGS. 11I to 11L, a graph at a lower part shows brain wave data acquired from the estimation target object F037. In each of FIGS. 11E to 11H, graphs in the middle show outputs at the time of inputting the brain wave data acquired from the estimation target object F037 to the models with the ID 007, ID 043, ID 126 and ID 141, respectively.
(1) The light-colored lines indicate the twenty pain scores; (2) the black line indicates ensemble average values of (1); and (3) the dark-colored line indicates results of 10-sec moving averages of (2). The twenty pain scores correspond to pieces of data for which the twenty standardization parameters are used, respectively.

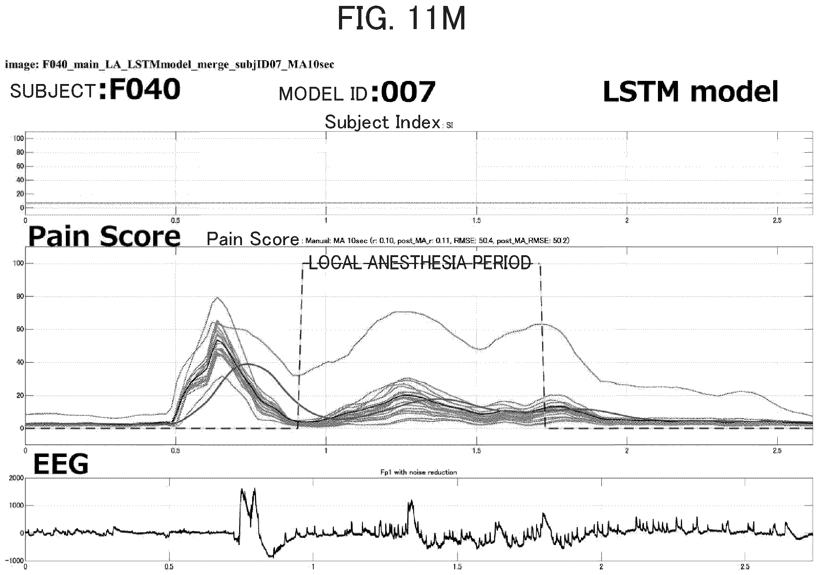

In each of FIGS. 11M to 11P, a graph at a lower part shows brain wave data acquired from the estimation target object F040. In each of FIGS. 11M to 11P, graphs in the middle show outputs at the time of inputting the brain wave data acquired from the estimation target object F040 to the models with the ID 007, ID 043, ID 126 and ID 141, respectively. (1) The light-colored lines indicate the twenty pain scores; (2) the black line indicates ensemble average values of (1); and (3) the dark-colored line indicates results of 10-sec moving averages of (2). The twenty pain scores correspond to pieces of data for which the twenty standardization parameters are used, respectively.

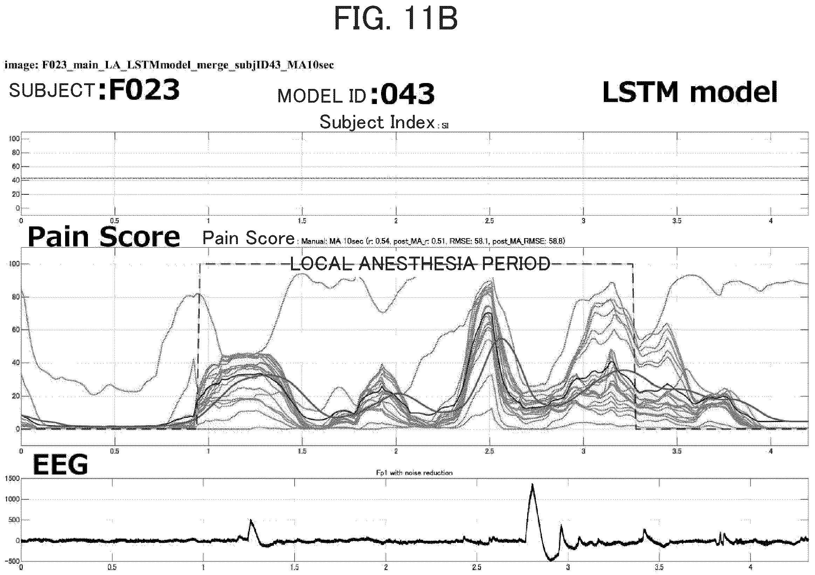

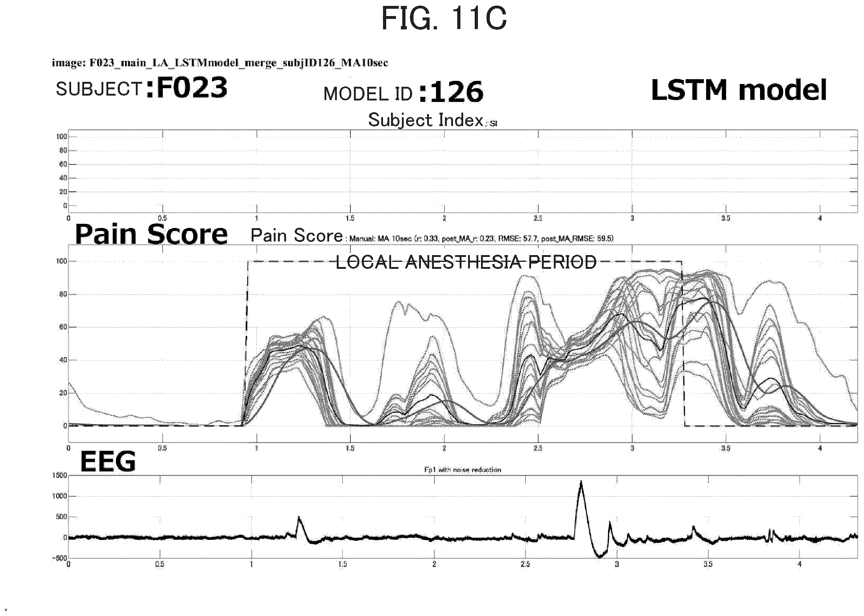

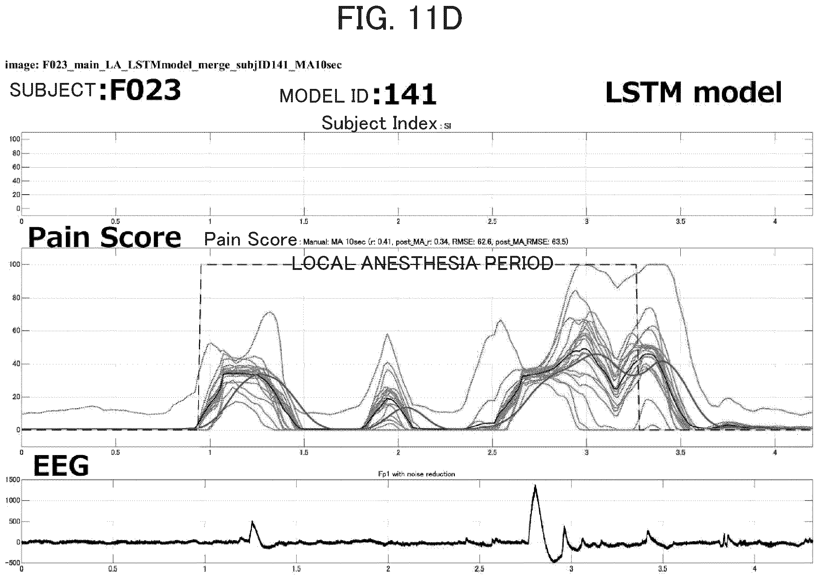

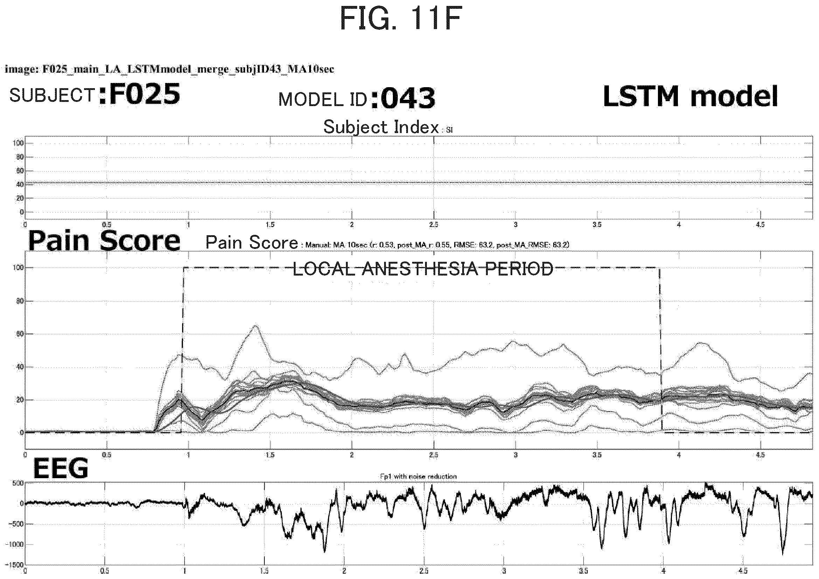

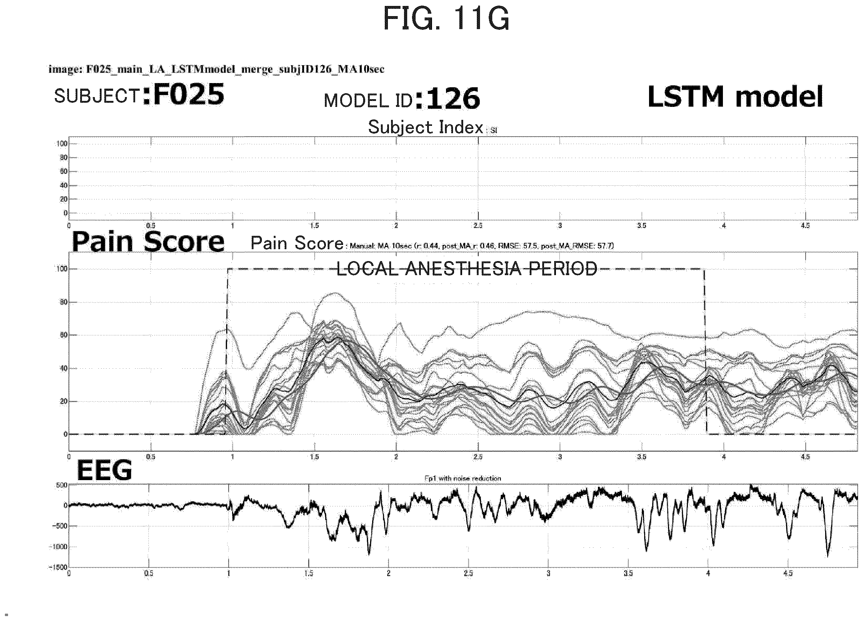

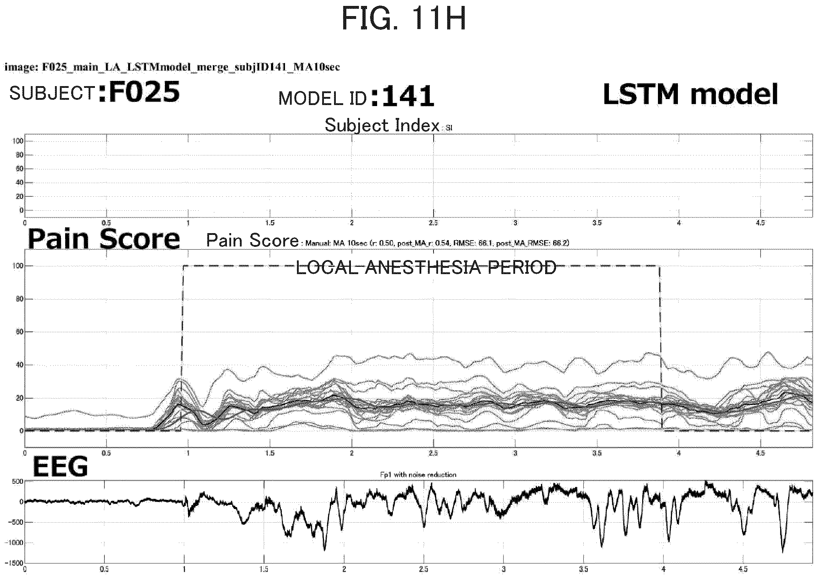

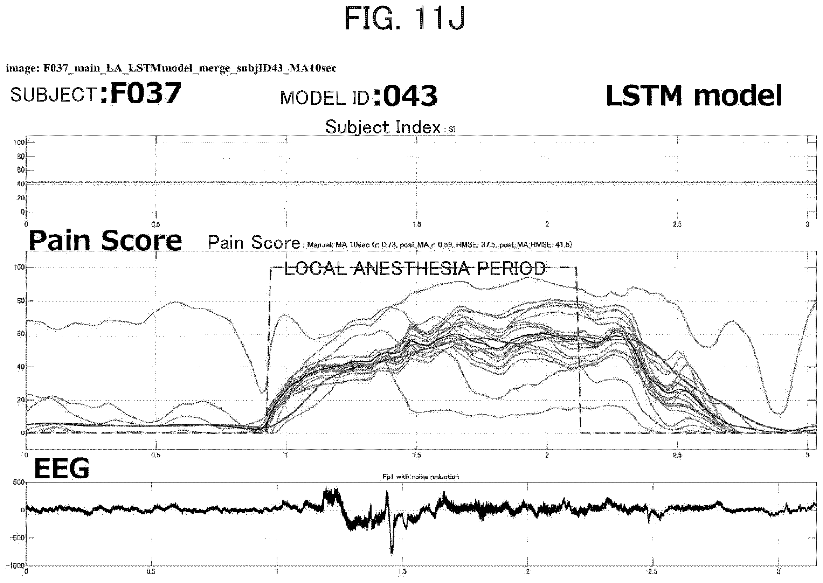

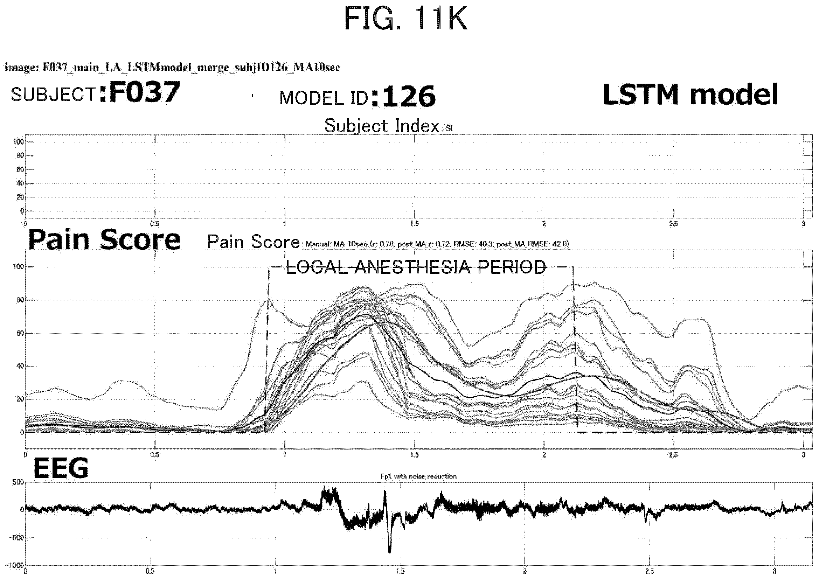

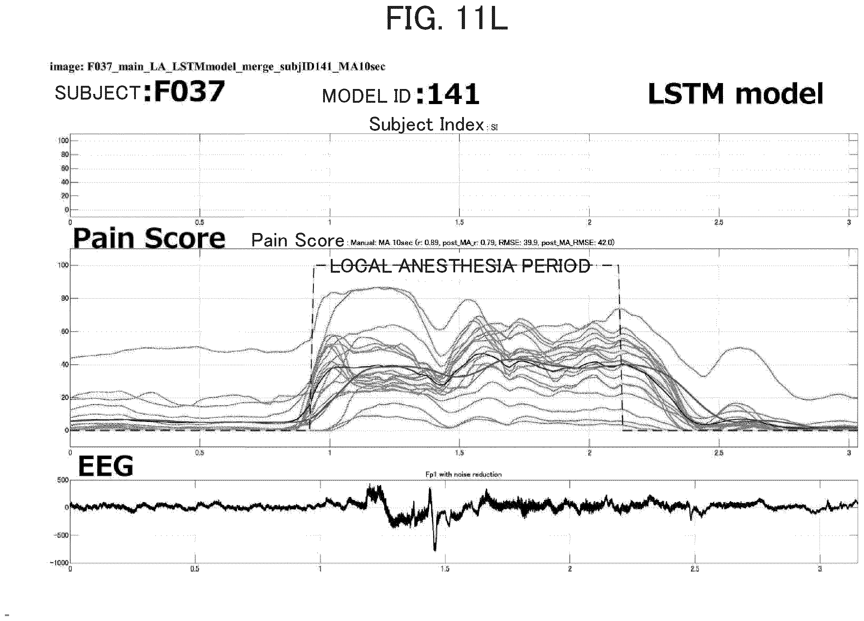

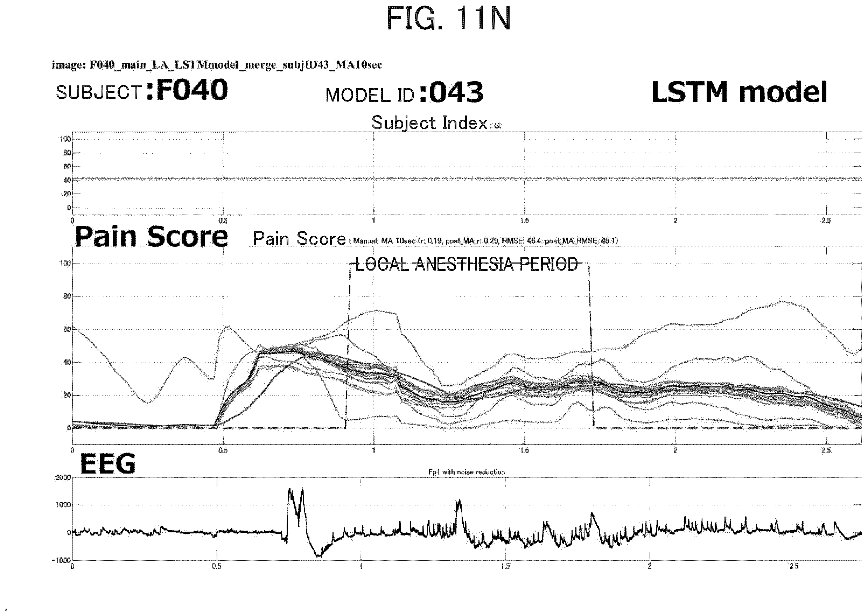

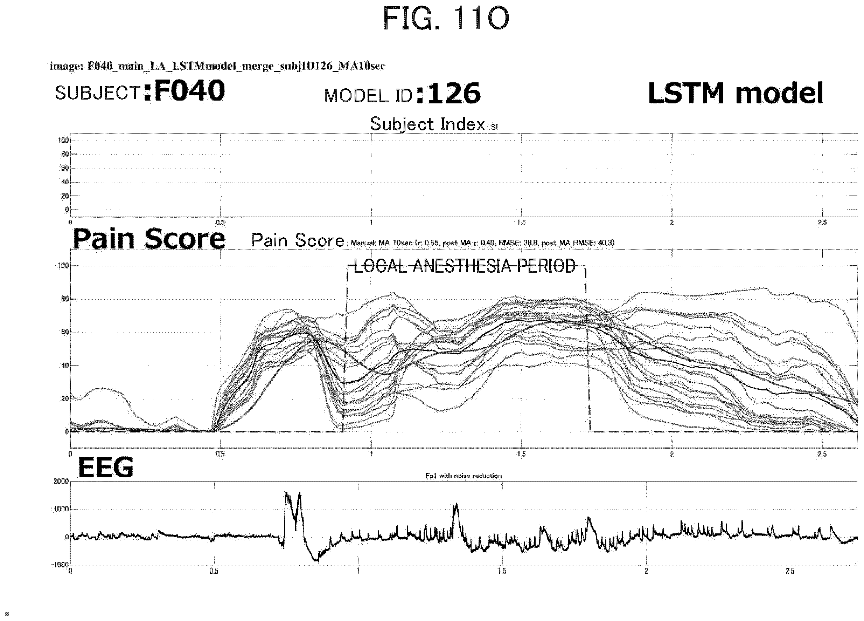

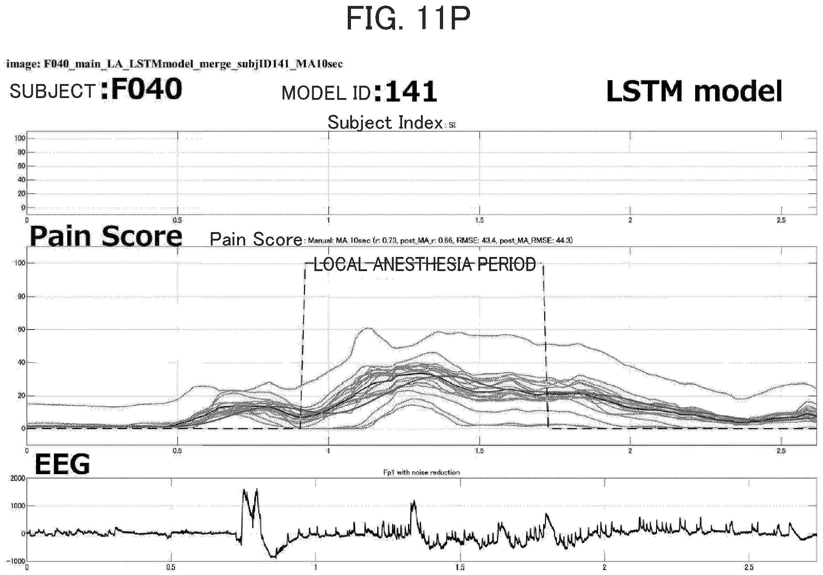

As seen from the results of FIGS. 11, the initial rising up of pain was almost the same though the models were different models. Further, in the different models, different time-series patterns of pain scores were displayed in the local anesthesia section. This can be thought to indicate that the model-specific detection power depends on a created personal model (functions as a personal filter). Further, the possibility that the accuracy can be increased by an addition average of a plurality of models is suggested.

Further, by using the method of the present disclosure, it is possible to highly accurately predict a pain that can be experienced by an estimation target object without giving a stimulus to the estimation target object in advance. Since giving a stimulus to an estimation target object is a burden on the estimation target object, it is useful that it is unnecessary to give a stimulus in advance.

As above, preferred embodiments of the present disclosure have been exemplified. It is understood that the scope of the present disclosure should be interpreted only by the claims. It is understood that the content of the patents, patent applications and other documents quoted in the present specification should be incorporated in the present specification by reference as if the content itself were specifically described in the present specification.

Further, though pain has been exemplified as an estimation target object in the embodiments described above, the estimation target object is not limited thereto. The estimation target object may be any object as long as it is a sensation the subjective assessment of which can be estimated, such as the sense of taste, the sense of smell, the sense of sight and the sense of touch.

### INDUSTRIAL APPLICABILITY

The present disclosure is useful in that a system and the like capable of estimating a subjective assessment made by an estimation target object can be provided. Further, the present disclosure is also useful in that it is possible to highly accurately predict a subjective assessment by an estimation target object without giving a load to the estimation target object in advance. Especially, the present disclosure can highly accurately predict a pain that can be experienced by an estimation target object without giving the pain to the estimation target object in advance.

### EXPLANATION OF REFERENCE NUMERALS

- 100:: System
- 110:: Reception means
- 120:: Processor
- 130:: Storage means
- 140:: Output means

## Claims

1. A system for estimating a subjective assessment made by an estimation target object, the system comprising:
a reception means that receives feature data of a biosignal acquired from the estimation target object;
a storage means that stores a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, and the plurality of models including a first model that has learned the first feature template and a second model that has learned the second feature template; and
an estimation means that estimates the subjective assessment made by the estimation target object, based on the feature data and the plurality of feature templates or the plurality of models.

2. The system according to claim 1, wherein
the estimation means is configured to perform:
obtaining a plurality of correlation coefficient sets by correlating each of the plurality of feature templates with the piece of feature data; and
estimating the subjective assessment made by the estimation target object, based on the plurality of correlation coefficient sets.

3. The system according to claim 2, wherein
the estimating of the subjective assessment by the estimation target object based on the plurality of correlation coefficient sets comprises:
identifying, for each of the plurality of correlation coefficient sets, a value indicating a subjective assessment that is associated with a sample corresponding to a highest correlation coefficient;
taking an ensemble average of the values indicating the subjective assessments of the plurality of correlation coefficient sets; and
determining a score indicating the subjective assessment based on the ensemble average.

4. The system according to claim 2, wherein
the estimating of the subjective assessment by the estimation target object based on the plurality of correlation coefficient sets comprises:
identifying, for each of the plurality of correlation coefficient sets, values indicating subjective assessments that are associated with samples corresponding to a top plurality of correlation coefficients;
obtaining an ensemble average correlation coefficient by, for each of the correlation coefficient sets, taking an ensemble average of the values indicating the top plurality of the subjective assessments;
taking an ensemble average of the ensemble average correlation coefficients of the plurality of correlation coefficient sets; and
determining a score indicating the subjective assessment based on the ensemble average.

5. The system according to claim 1, wherein
the estimation means is configured to perform:
obtaining a plurality of outputs by inputting the feature data to each of the plurality of models, the plurality of outputs including a first output outputted from the first model and a second output outputted from the second model; and
estimating the subjective assessment made by the estimation target object, based on the plurality of outputs.

6. The system according to claim 5, wherein
the estimating of the subjective assessment by the estimation target object based on the plurality of outputs comprises:
taking an ensemble average of the plurality of outputs; and
determining a score indicating the subjective assessment based on the ensemble average.

7. The system according to claim 5 or 6, wherein
the storage means further stores a plurality of standardization parameters extracted from the plurality of biosignals, the plurality of standardization parameters including a plurality of first standardization parameters extracted from the first biosignal acquired from the first modeling target object and a plurality of second standardization parameters extracted from a plurality of second biosignals acquired from the second modeling target object,
the estimation means Is configured to further perform
generating a plurality of pieces of standardized feature data by standardizing the feature data by the plurality of standardization parameters, the plurality of pieces of standardized feature data including a plurality of pieces of first standardized feature data obtained by standardizing the feature data by the plurality of first standardization parameters, and a plurality of pieces of second standardized feature data obtained by standardizing the feature data by the plurality of second standardization parameters, and
the obtaining of the plurality of outputs by inputting the feature data to each of the plurality of models comprises
obtaining a plurality of outputs of the plurality of models by inputting the plurality of pieces of standardized feature data to the plurality of models, the plurality of outputs of the plurality of models including a plurality of first outputs obtained by inputting the plurality of pieces of first standardized feature data to the first model and a plurality of second outputs obtained by inputting the plurality of pieces of second standardized feature data to the second model.

8. The system according to claim 7, wherein
the estimating of the subjective assessment by the estimation target object based on the plurality of outputs comprises:
obtaining a plurality of ensemble average outputs by taking an ensemble average of the plurality of outputs of each of the plurality of models, the plurality of ensemble average outputs including a first ensemble average output obtained by taking an ensemble average of the plurality of first outputs and a second ensemble average output obtained by taking an ensemble average of the plurality of second outputs;
taking an ensemble average of the plurality of ensemble average outputs; and
determining a score indicating the subjective assessment based on the ensemble average.

9. The system according to any one of claims 1 to 8, wherein
the reception means receives no-load feature data of a biosignal when a load is not given to the estimation target object, and
the estimation means is configured to perform:
selecting at least one of the plurality of feature templates to be used to estimate the subjective assessment by the estimation target object or at least one of the plurality of models to be used to estimate the subjective assessment by the estimation target object, based on the no-load feature data; and
estimating the subjective assessment made by the estimation target object, based on the feature data and the at least one of the plurality of the feature templates or the at least one of the plurality of models that has been selected.

10. The system according to any one of claims 1 to 9, wherein
the plurality of modeling target objects are n modeling target objects, the plurality of feature templates are n feature templates, the plurality of models are n models, and n is an integer equal to or larger than two.

11. The system according to any one of claims 1 to 10, wherein
the biosignal acquired from the estimation target object is a biosignal at a time when a stimulus is given to the estimation target object,
the plurality of biosignals are a plurality of biosignals at a time when a stimulus is given to the plurality of modeling target objects, the first biosignal is a first biosignal at the time when the stimulus is given to the first modeling target object, and the second biosignal is a second biosignal at the time when the stimulus is given to the second modeling target object, and
the estimation means estimates a pain experienced by the estimation target object.

12. A method for estimating a subjective assessment made by an estimation target object, the method being executable by a computer comprising a storage means, the storage means storing a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, the plurality of models including a first model that has learned the first feature template and a second model that has learned the second feature template, the method comprising:
receiving feature data of a biosignal acquired from the estimation target object; and
estimating the subjective assessment made by the estimation target object, based on the feature data and the plurality of feature templates or the plurality of models.

13. A program for estimating a subjective assessment made by an estimation target object, the program being executable by a computer comprising a processor unit, the program being executable by a computer system comprising a storage means, the storage means storing a plurality of feature templates extracted from a plurality of biosignals acquired from a plurality of modeling target objects including a first modeling target object and a second modeling target object, or a plurality of models that have learned the plurality of feature templates, each of the plurality of feature templates associating pieces of feature data of a plurality of samples sampled from a biosignal with values indicating subjective assessments, the plurality of feature templates including a first feature template extracted from a first biosignal acquired from the first modeling target object and a second feature template extracted from a second biosignal acquired from the second modeling target object, each of the plurality of models being configured to output a value indicating a subjective assessment in response to an input of feature data, the plurality of models including a first model that has learned the first feature template and a second model that has learned the second feature template,
the program causing the processor unit to perform a process comprising:
receiving feature data of a biosignal acquired from the estimation target object; and
estimating the subjective assessment made by the estimation target object, based on the feature data and the plurality of feature templates or the plurality of models.
